# EUROPÄISCHE PATENTSCHRIFT

(11) **EP 0 958 291 B1**
(45) Veröffentlichungstag und Bekanntmachung des Hinweises auf die Patenterteilung: **21.01.2009**
(21) Anmeldenummer: 98905274.1
(22) Anmeldetag: 08.01.1998
(51) Int. Cl.: C07D 413/10, A01N 43/72, C07D 498/10, C07D 417/10, C07D 261/04, C07D 291/04, C07D 273/00, C07D 263/10, C07D 261/20, C07D 277/10, C07D 277/34

(54) **3-HETEROXYCLYL-SUBSTITUIERTE BENZOYLDERIVATE**
3-HETEROCYCLYL-SUBSTITUTED BENZOYL DERIVATIVES
DERIVES DE BENZOYLE SUBSTITUES PAR 3-HETEROCYCLYLE

(30) Priorität: 17.01.1997 DE 19701446
(43) Veröffentlichungstag der Anmeldung: 24.11.1999
(73) Patentinhaber: BASF SE, 67056 Ludwigshafen (DE)
(72) Erfinder: VON DEYN, Wolfgang, D-67435 Neustadt (DE); HILL, Regina, Luise, D-67346 Speyer (DE); KARDORFF, Uwe, D-68159 Mannheim (DE); BAUMANN, Ernst, D-67373 Dudenhofen (DE); ENGEL, Stefan, D-65510 Idstein (DE); MAYER, Guido, D-67433 Neustadt (DE); WITSCHEL, Matthias, D-67061 Ludwigshafen (DE); RACK, Michael, D-69123 Heidelberg (DE); GÖTZ, Norbert, D-67547 Worms (DE); GEBHARDT, Joachim, D-67157 Wachenheim (DE); MISSLITZ, Ulf, D-67433 Neustadt (DE); WALTER, Helmut, D-67283 Obrigheim (DE); WESTPHALEN, Karl-Otto, D-67346 Speyer (DE); OTTEN, Martina, D-67069 Ludwigshafen (DE); RHEINHEIMER, Joachim, D-67063 Ludwigshafen (DE)
(86) Internationale Anmeldenummer: PCT/EP1998/000069
(87) Internationale Veröffentlichungsnummer: WO 1998/031681

(56) Entgegenhaltungen:
- EP-A- 0 203 428
- WO-A-96/26206
- DE-A- 2 513 750
- FR-A- 2 316 235

## Beschreibung

Die vorliegende Erfindung betrifft 3-Heterocyclyl-substituierte Benzoylderivate der Formel I 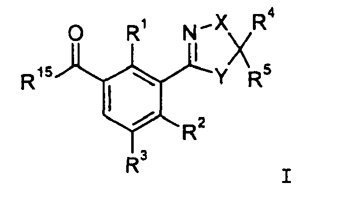 in der die Variablen folgende Bedeutungen haben:
- R¹: Nitro, C₁-C₆-Alkyl, C₁-C₆-Alkoxy, C₁-C₆-Halogenalkyl, C₁-C₆-Alkylsulfonyl,C₁-C₆-Halogenalkylsulfonyl ;
- R²: Nitro, Halogen, C₁-C₆-Alkyl, C₁-C₆-Halogenalkyl, C₁-C₆-Alkylthio, C₁-C₆-Alkylsulfinyl, C₁-C₆-Alkylsulfonyl, C₁-C₆-Halogenalkylsulfonyl;
- R³: Wasserstoff;
- R⁴, R⁵: Wasserstoff;
- X: O;
- Y: CR¹3R¹⁴;
- R¹³, R¹⁴: Wasserstoff, C₁-C₄-Alkyl, C₁-C₄-Halogenalkyl, C₁-C₄-Alkoxycarbonyl, C₁-C₄-Halogenalkoxycarbonyl, CONR⁷R⁸, mit R⁷ in der Bedeutung von Wasserstoff oder C₁-C₄-Alkyl und R⁸ in der Bedeutung von C₁-C₄-Alkyl;
- R¹⁵: ein in 4-Stellung verknüpftes Pyrazol der Formel II 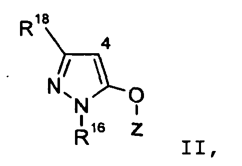 wobei
R¹⁶ C₁-C₆-Alkyl;
Z H;
R¹⁸ H; bedeuten;
sowie deren landwirtschaftlich brauchbaren Salze.

### Ebenso betrifft die Erfindung

### 3-Heterocyclyl-substituierte Benzoylderivate der Formel Ib23,

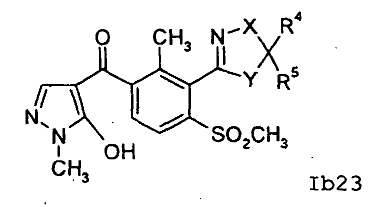

in der die Variablen folgende Bedeutungen haben:

| Nr. | X | R⁴ | R⁵ | Y |
|---|---|---|---|---|
| Ib23.1 | CH₂ | H | CH₃ | O |
| Ib23.2 | CH₂ | H | H | O |
| Ib23.3 | C (CH₃) ₂ | H | H | O |
| Ib23.4 | CH₂ | H | C₂H₅ | O |
| Ib23.5 | CH₂ | CH₃ | CH₃ | O |
| Ib23.6 | CH (CH₃) | H | CH₃ | O |
| Ib23.7 | CH(C₂H₅) | H | CH₃ | O |
| Ib23.8 | CH[CH(CH₃)₂] | H | H | O |
| Ib23.9 | CH₂ | H | CH (CH₃)₂ | O |
| Ib23.10 | CH(C₂H₅) | H | C₂H₅ | O |
| Ib23.11 | -CH- (CH₂) ₄- | | H | O |
| Ib23.12 | C=O | CH₃ | CH₃ | O |
| In23.13 | C=O | H | C₂H₅ | O |
| Ib23.14 | C=O | C₂H₅ | C₂H₅ | O |
| Ib23.15 | C=O | H | H | O |
| Ib23.16 | C=O | H | CH₃ | O |
| Ib23.17 | CH₂ | H | CH₃ | S |
| Ib23.18 | CH₂ | H | H | S |
| Ib23.19 | C (CH₃) ₂ | H | H | S |
| Ib23.20 | CH₂ | H | C₂H₅ | S |
| In23.21 | CH₂ | CH₃ | CH₃ | S |
| Ib23.22 | CH (CH₃) | H | CH₃ | S |
| Ib23.23 | CH (C₂H₅) | H | CH₃ | S |
| Ib23.24 | CH (C₂H₅) | H | C₂H₅ | S |
| Ib23.25 | -CH- (CH₂)₄- | | H | S |
| Ib23.26 | CH[CH(CH₃)₂] | H | H | S |
| Ib23.27 | CH₂ | H | CH (CH₃) ₂ | S |
| Ib23.28 | CH₂ | H | CH₃ | NH |
| Ib23.29 | CH₂ | H | H | NH |
| Ib23.30 | C(CH3)₂ | H | H | NH |
| Ib23.31 | CH₂ | H | C₂H₅ | NH |
| Ib23.32 | CH₂ | CH₃ | CH₃ | NH |
| Ib23.33 | CH(CH₃) | H | CH₃ | NH |
| Ib23.34 | CH(C₂H₅) | H | CH₃ | NH |
| Ib23.35 | CH(C₂H₅) | H | C₂H₅ | NH |
| Ib23.36 | -CH-(CH₂)₄- | | H | NH |
| Ib23.37 | CH [CH (CH₃)₂] | H | H | NH |
| Ib23.38 | CH₂ | H | CH (CH₃)₂ | NH |
| Ib23.39 | CH₂ | H | CH₃ | NCH₃ |
| Ib23.40 | CH₂ | H | H | NCH₃ |
| Ib23.41 | C(CH₃)₂ | H | H | NCH₃ |
| Ib23.42 | CH₂ | H | C₂H₅ | NCH₃ |
| Ib23.43 | CH₂ | CH₃ | CH₃ | NCH₃ |
| Ib23.44 | CH(CH₃) | H | CH₃ | NCH₃ |
| Ib23.45 | CH(C₂H₅) | H | CH₃ | NCH₃ |
| Ib23.46 | CH[CH(CH₃)₂] | H | H | NCH₃ |
| Ib23.47 | CH₂ | H | CH(CH₃)₂ | NCH₃ |
| Ib23.48 | CH (C₂H₅) | H | C₂H₅ | NCH₃ |
| Ib23.49 | -CH-(CH₂)₄- | | H | NCH₃ |
| Ib23.50 | CH₂ | H | CH₃ | NC₂H₅ |
| Ib23.51 | CH₂ | H | H | NC₂H₅ |
| Ib23.52 | C (CH₃) ₂ | H | H | NC₂H₅ |
| Ib23.53 | CH₂ | H | C₂H₅ | NC₂H₅ |
| Ib23.54 | CH₂ | CH₃ | CH₃ | NC₂H₅ |
| Ib23.55 | CH(CH₃) | H | CH₃ | NC₂H₅ |
| Ib23.56 | CH (C₂H₅) | H | CH₃ | NC₂H₅ |
| Ib23.57 | CH [CH (CH₃)₂] | H | H | NC₂H₅ |
| Ib23.58 | CH₂ | H | CH (CH₃)₂ | NC₂H₅ |
| Ib23.59 | CH(C₂H₅) | H | C₂H₅ | NC₂H₅ |
| Ib23.60 | -CH- (CH₂)₄- | | H | NC₂H₅ |
| Ib23.61 | CH₂ | =O | | S |
| Ib23.62 | CH (CH₃) | =O | | S |
| Ib23.63 | CH (C₂H₅) | =O | | S |
| Ib23.64 | CH [CH (CH₃)₂] | =O | | S |
| Ib23.65 | C (CH₃)₂ | =O | | S |
| Ib23.66 | CCH₃(C₂H₅) | =O | | S |
| Ib23.67 | CCH₃ [CH (CH₃)₂] | =O | | S |
| Ib23.68 | CH₂ | =O | | NH |
| Ib23.69 | CH (CH₃) | =O | | NH |
| Ib23.70 | CH (C₂H₅) | =O | | NH |
| Ib23.71 | CH [CH (CH₃) ₂] | =O | | NH |
| Ib23.72 | C (CH₃)₂ | =O | | NH |
| Ib23.73 | CCH₃ (C₂H₅) | =O | | NH |
| Ib23.74 | CCH₃ [CH (CH₃) ₂] | =O | | NH |
| Ib23.75 | CH₂ | =O | | NCH₃ |
| Ib23.76 | CH(CH₃) | =O | | NCH₃ |
| Ib23.77 | CH(C₂H₅) | =O | | NCH₃ |
| In23.78 | CH [CH(CH₃) ₂] | =O | | NCH₃ |
| Ib23.79 | C(CH₃)₂ | =O | | NCH₃ |
| Ib23.80 | CCH₃(C₂H₅) | =O | | NCH₃ |
| Ib23.81 | CCH₃[CH(CH₃)₂] | =O | | NCH₃ |
| Ib23.82 | O | COOCH₃ | H | CH₂ |
| Ib23.83 | O | COOC₂H₅ | H | CH₂ |
| Ib23.84 | O | CONHCH₃ | H | CH₂ |
| Ib23.85 | O | CON(CH₃)₂ | H | CH₂ |
| Ib23.86 | O | CONHC₂H₅ | H | CH₂ |
| Ib23.87 | O | CON(C₂H₅)₂ | H | CH₂ |
| Ib23.88 | O | CH₃ | H | CH₂ |
| Ib23.89 | O | C₂H₅ | H | CH₂ |
| Ib23.90 | O | CH (CH₃) ₂ | H | CH₂ |
| Ib23.91 | O | COC₂H₅ | H | CH₂ |
| Ib23.92 | O | CH₂CN | H | CH₂ |
| Ib23.93 | O | CH₂N(CH₃)₂ | H | CH₂ |
| Ib23.94 | O | CH₂ON=C(CH₃) ₂ | H | CH₂ |
| Ib23.95 | O | CH(OC₂H₅)₂ | H | CH₂ |
| Ib23.96 | O | CH(OCH₃)₂ | H | CH₂ |
| Ib23.97 | O | CH₃ | CH₃ | CH₂ |
| Ib23.98 | O | CH₃ | C₂H₅ | CH₂ |
| Ib23.99 | O | C₂H₅ | C₂H₅ | CH₂ |
| Ib23.100 | O | - (CH₂)₄- | | CH₂ |
| Ib23.101 | O | -(CH₂)₂-O-(CH₂)₂- | | CH₂ |
| Ib23.102 | O | H | - (CH₂)₃-CH- | |
| Ib23.103 | O | H | - (CH₂)₄-CH- | |
| Ib23.104 | O | CH₃ | H | CHCH₃ |
| Ib23.105 | O | H | H | CH₂ |
| Ib23.106 | S | =O | | O |
| Ib23.107 | CH₂ | =S | | S |
| Ib23.108 | CH (CH₃) | =S | | S |
| Ib23.109 | CH (C₂H₅) | =S | | S |
| Ib23.110 | C (CH₃)₂ | =S | | S |
| Ib23.111 | O | =O | | NH |
| Ib23.112 | O | =O | | NCH₃ |
| Ib23.113 | O | CH₃ | H | NH |
| Ib23.114 | O | C₂H₅ | H | NH |
| Ib23.115 | O | CH₃ | CH₃ | NH |
| Ib23.116 | O | C₂H₅ | C₂H₅ | NH |
| Ib23.117 | O | CH₃ | H | NCH₃ |
| Ib23.118 | O | C₂H₅ | H | NCH₃ |
| Ib23.119 | O | CH₃ | CH₃ | NCH₃ |
| Ib23.120 | O | C₂H₅ | C₂H₅ | NCH₃ |
| Ib23.121 | NH | =O | | NH |
| Ib23.122 | NH | =O | | NCH₃ |
| Ib23.123 | NCH₃ | =O | | NH |
| Ib23.124 | NCH₃ | =O | | NCH₃ |
| Ib23.125 | NC₂H₅ | =O | | NH |
| Ib23.126 | NC₂H₅ | =O | | NC₂H₅ |

sowie deren landwirtschaftlich brauchbaren Salze.

Außerdem betrifft die Erfindung Verfahren und Zwischenprodukte zur Herstellung von Verbindungen der Formel I /Ib23, Mittel welche diese enthalten sowie die Verwendung dieser Derivate oder diese enthaltende Mittel zur Schadpflanzenbekämpfung.

Aus der Literatur, beispielsweise aus WO 96/26206 sind Pyrazol-4-yl-benzoylderivate bekannt, die in 3-Position einen 5- oder 6-gliedrigen heterocyclischen, gesättigten oder ungesättigten Rest, enthaltend ein bis drei Heteroatome, ausgewählt aus der Gruppe O, S oder N tragen.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 3- Heterocyclyl -substituierten Benzoylderivate der Formel I/Ib 23 sowie deren herbizide Wirkung gefunden.

Ferner wurden herbizide Mittel gefunden, die die Verbindungen I/lb23. enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I /lb23 gefunden.

Die Verbindungen der Formel I/lb23 können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I/lb23 können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I/lb23 nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹⁸ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyls Cyanoalkyl-, Alkoxy-, Halogenalkoxy-, Alkyliminooxy-, Alkylcarbonyloxy, Alkylthio-, Halogenalkylthio-, Alkylsulfinyl-, Halogenalkylsulfinyl-, Alkylsulfonyl-, Halogenalkylsulfonyl-, Alkoxycarbonyl-, Halogenalkoxycarbonyl, Alkenyloxy-, Alkinyloxy-, Dialkylamino-, Dialkylhydrazino-, Alkoxyalkyl-, Hydroxyalkoxyalkyl, Dialkoxyalkyl-, Alkylthioalkyl-, Dialkylaminoalkyl-, Dialkylhydrazinoalkyl-, Alkyliminooxyalkyl-, Alkoxycarbonylalkyl- und Alkoxyalkoxy-Teile können geradkettig oder verzweigt sein. Sofern sicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: sowie die Alkylteile von Di- (C₁-C₄-alkoxy)-C₁-C₄-alkyl, [2,2-Di-(C₁-C₄-alkyl)-hydrazino-1]-C₁-C₄-alkyl, C₁-C₆₋Alkyliminooxy-C₁-C₄-alkyl, Hydroxy-C₁-C₄-alkoxy-C₁-C₄-alkyl und C₁-C₄-Alkylcarbonyloxy: z.B. Methyl, Ethyl, Propyl, 1-Methylethyl, Butyl, 1-Methylpropyl, 2-Methylpropyl und 1,1-Dimethylethyl;
- C₁-C₆-Alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy: sowie die Alkoxyteile von Di- (C₁-C₄-alkoxy) C₁-C₄-alkyl und Hydroxy-C₁-C₄-alkoxy-C₁-C₄-alkyl, z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio. 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-l-methylpropylthio und 1-Ethyl-2-methylpropylthio; C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl. 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
- C₁-C₆-Alkylsulfonyl (C₁-C₆-Alkyl-S(=O)₂-): z.B. Methyl-sulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylbentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl; 2-Chlorethylsulfonyl, bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsuifonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl. 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl und 1,1-Dimethoxycarbonyl;
- C₁-C₄-Halogerialkoxycarbonyl: einen C₁-C₄-Alkoxycarbonylrest wie voranstehend genannt, der partiell oder vollständig durch Fluor, Chlor, Brom und/oder Iod substituiert ist, also z.B. Fluormethoxycarbonyl, Difluörmethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxy-carbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl und 4-Iodbutoxycarbonyl;
- Di-(C₁-C₄-alkyl)-amino- z.B. N,N-Dimethylamino, N,N-Diethyl-amino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino. N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;

Besonders außerordentlich bevorzugt sind 3-Heterocyclyl-substituierte Benzoylderivate der Formel I, wobei
- R¹: Nitro, C₁-C₆-Alkyl, wie z.B. Methyl und Ethyl, C₁-C₆-Alkoxy, wie z.B. Methoxy und Ethoxy, C₁-C₆-Halogenalkyl wie z.B. Difluor-methyl und Trifluormethyl, C₁-C₆-Alkyl-sulfonyl, wie z.B. Methylsulfonyl, Ethyl-sulfonyl und Propylsulfonyl oder C₁-C₆-Halogenalkylsulfonyl, z.B. Trifluor-methylsulfonyl und Pentafluorethylsulfonyl;
bedeutet.

Ebenso besonders außerordentlich bevorzugt sind 3-Heterocyclyl-substituierte Benzoylderivate der Formel I, wobei
- R²: Nitro, Halogen, wie z.B. Chlor und Brom, C₁-C₆-Alkyl, wie z.B. Methyl und Ethyl, C₁-C₆-Halogenalkyl, wie z.B. Difluormethyl und Trifluormethyl, C₁-C₆-Alkylthio wie z.B. Methylthio und Ethylthio, C₁-C₆-Alkyl-sulfinyl, wie z.B. Methylsulfinyl und Ethyl-sulfinyl, C₁-C₆-Alkylsulfonyl, wie z.B. Methylsulfonyl, Ethylsulfonyl und Propyl-sulfonyl oder C₁-C₆-Halogenalkylsulfonyl, wie z.B. Trifluomethylsulfonyl und Penta-fluorethylsulfonyl;
bedeuten.

Ebenso insbesondere bevorzugt sind 3-Hetrocyclyl-substituierte Benzoylderivate der Formel I, in der die Variablen folgende Bedeutungen haben:
- R¹: C₁-C₆-Alkyl oder C₁-C₆-Alkyl-sulfonyl; insbesondere Methyl oder Methyl-sulfonyl;
- R²: Nitro, Halogen, C₁-C₆-Alkylthio, C₁-C₆-Alkyl-sulfinyl oder C₁-C₆-Alkylsulfonyl; insbesondere Nitro, Chlor, Methylthio, Methylsulfinyl, Methylsulfonyl, Ethylsulfonyl oder Propylsulfonyl;
- R³: Wasserstoff;
- R⁴, R⁵: Wasserstoff,
- X: O;
- Y: CR¹³R¹⁴; R¹³, R¹⁴ Wasserstoff, C₁-C₄-Alkyl oder C₁-C₄-Halogenalkyl; insbesondere Wasserstoff, Methyl oder Chlormethyl;
- R¹⁶: C₁-C₆-Alkyl; insbesondere Methyl, Ethyl, Propyl, 2-Methylpropyl oder Butyl;
- Z: H;
sowie deren landwirtschaftlich brauchbaren Salze; insbesondere Alkalimetallsalze und Ammoniumsalze.

Die 3-Heterocyclyl-substituierten Benzolylderivate der Formel I /lb23 sind auf verschiedene Art und Weise erhältlich, beispielsweise nach folgendem Verfahren:

### Verfahren A:

Umsetzung von Pyrazolen der Formel II (mit Z = H) mit einer aktivierten Benzoesäure IIIa oder einer Benzoesäure IIIβ, die vorzugsweise in situ aktiviert wird, zu dem Acylierungsprodukt und anschließende Umlagerung.

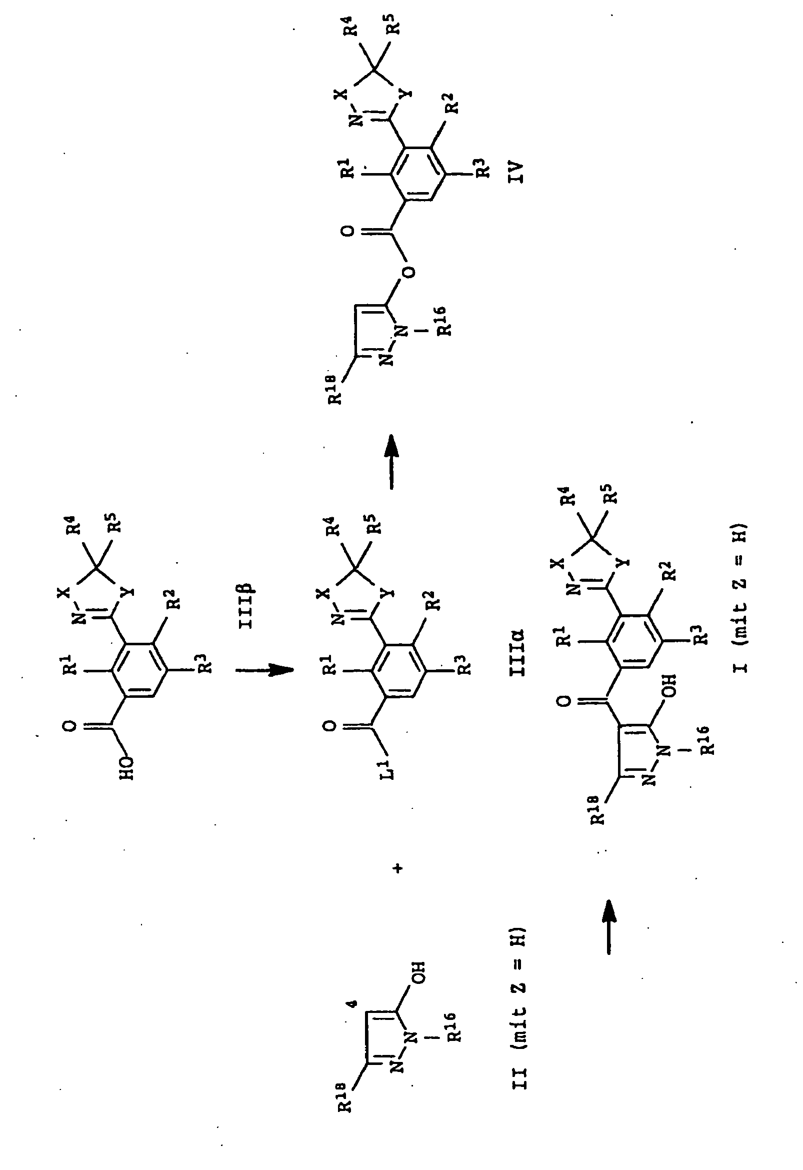

L¹ steht für eine nucleophil verdrängbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Benzoesäure kann direkt eingesetzt werden, wie im Fall der Benzoylhalogenide oder in situ erzeugt werden, z.B. mit

Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamin, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0 - 10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 40°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 und US 4 643 757 genannt).

### verfahren B: (nicht erfindungsgemäß)

Umsetzung von 3-Heterocyclyl-substituierten Benzoylderivaten der Formel I (mit Z = H) mit einer Verbindung der Formel V (mit Z = SO₂R¹⁷):

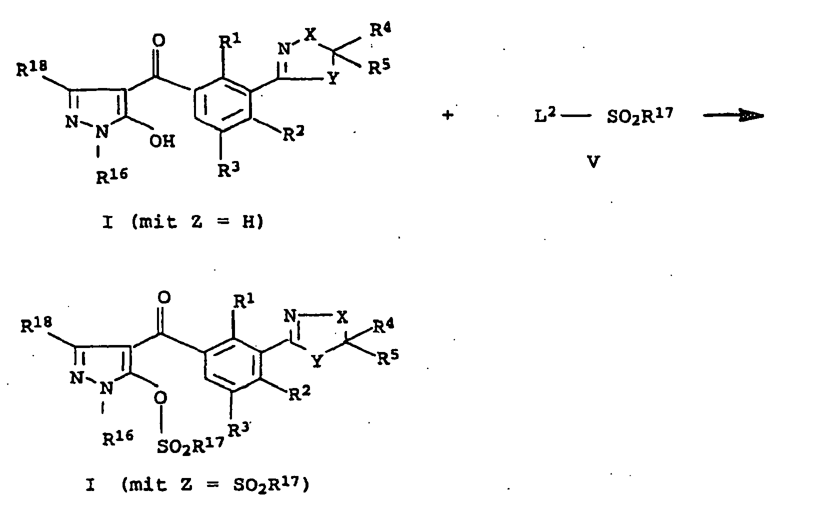

L² steht für eine nucleophil verdrängbare Abgangsgruppe, wie Halogen, z.B. Brom, Chlor, Hetaryl, z.B. Imidazolyl, Pyridyl, Sulfonat, z.B. OSO₂R¹⁷.

Die Verbindungen der Formel V können direkt eingesetzt werden, wie z.B. im Fall der Sulfonsäurehalogenide, Sulfonsäureanhydride oder in situ erzeugt werden, z.B. aktivierte Sulfonsäuren (mittels Sulfonsäure und Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf II, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin und Pyridin.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II (mit Z = H) sind bekannt oder können nach an sich bekannten Verfahren hergestellt werden (z.B. EP-A 240 001 und J. Prakt. Chem. 315, 383 (1973)).

3-Heterocyclyl-substituierte Benzoesäurederivate der Formel III sind neu, 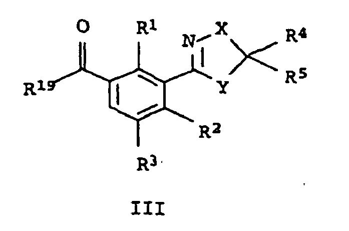 wobei die Variablen folgende Bedeutung haben.
- R¹;: Nitro, C₁-C₆-Alkyl, C₁-C₆-Halogenalkyl, C₁-C₆-Alkoxy, C₁-C₆-Alkylsulfonyl oder C₁-C₆-Halogenalkylsulfonyl;
- R²: Nitro. Halogen, C₁-C₆-Alkyl, C₁-C₆-Halogenalkyl, C₁-C₆-Alkylthio, C₁-C₆-Alkylsulfinyl, C₁-C₆-Alkylsulfonyl, C₁-C₆-Halogenalkylsulfonyl;
- R³: Wasserstoff;
- R⁴, R⁵: Wasserstoff;
- R⁷: Wasserstoff oder C₁-C₄-Alkyl;
- R⁸: C₁-C₄-Alkyl;
- X: O;
- Y: CR¹³R¹⁴; R¹³, R¹⁴ Wasserstoff, C₁-C₄-Alkyl, C₁-C₄-Halogenalkyl, C₁-C₄-Alkoxycarbonyl, C₁-C₄-Halogenalkoxycarbonyl oder CONR⁷R⁸;
- R¹⁹: Hydroxy oder abhydrolysierbarer Rest;

Beispiele für abhydrolysierbare Reste sind Alkoxy-, Phenoxy-, Alkylthio-, Phenylthio-Reste, die gegebenenfalls substituiert sein können, Halogenide, Hetarylreste, die über Stickstoff gebunden sind, Amino, Imino-Reste, die gegebenenfalls substituiert sein können etc.

Bevorzugt sind 3-Heterocyclyl-substituierte Benzoesäurehalogenide der Formel IIIα', mit L^{1'} = Halogen ( ≙ III mit R¹⁹ = Halogen), 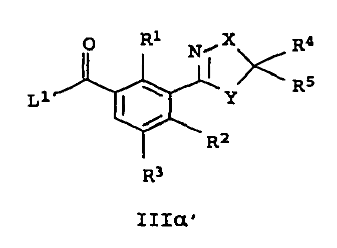 wobei die Variablen R¹ bis R⁵, X und Y die unter Formel III genannte Bedeutung haben und
- L^{1'}: Halogen, insbesondere Chlor oder Brom bedeutet.

Ebenso bevorzugt sind 3-Heterocyclyl-substituierte Benzoesäuren der Formel IIIβ ( ≙ III mit R¹⁹ = Hydroxy), 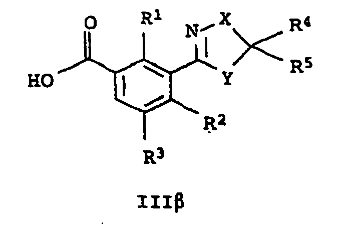 wobei die Variablen R¹ bis R⁵, X und Y die unter Formel III genannte Bedeutung haben.

Ebenso bevorzugt sind 3-Heterocyclyl-substituierte Benzoesäureester der Formel IIIγ ( ≙ III mit R¹⁹ = C₁-C₆-Alkoxy), 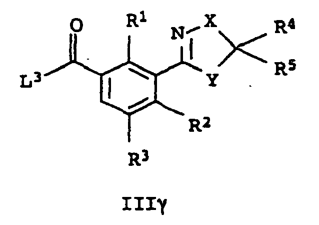 wobei die Variablen R¹ bis R⁵, X und Y die unter Formel III genannte Bedeutung haben und
- L³: C₁-C₆-Alkoxy bedeutet.

Die besonders bevorzugten Ausführungsformen der 3-Heteroacyclylsubstituierten Benzoesäurederivate der Formel III in Bezug auf die Variablen R¹ bis R⁵, X und Y entsprechen denen der 3-Hetero-cyclyl-substituierten Benzoylderivate der Formel I/lb23.

Ebenso bevorzugt sind 3-Heterocyclyl-substituierte Benzoesäurederivate der Formel III, in der die Variablen folgende Bedeutungen haben:
- R¹: C₁-C₆-Alkyl, C₁-C₆-Alkyl-sulfonyl; insbesondere Methyl; oder Methyl-sulfonyl;
- R²: Nitro, Halogen, C₁-C₆-Alkylthio, C₁-C₆-Alkyl-sulfinyl oder C₁-C₆-Alkylsulfonyl; insbesondere Nitro, Chlor, Methylthio, Methylsulfinyl, Methylsulfonyl, Ethylsulfonyl oder Propylsulfonyl; außerordentlich bevorzugt Chlor, Methylthio, Methylsulfonyl, Ethylsulfonyl oder Propylsulfonyl;
- R³: Wasserstoff;
- R⁴, R⁵: Wasserstoff,
- X: O;
- Y: CR¹³R¹⁴ ; R¹³, R¹⁴ Wasserstoff, C₁-C₄-Alkyl, C₁-C₄-Halogenalkyl oder C₁-C₄-Alkoxycarbonyl; insbesondere Wasserstoff, Methyl, Chlormethyl oder Methoxycarbonyl;
- R¹⁹: Hydroxy, Halogen oder C₁-C₆-Alkoxy; insbesondere Hydroxy, Chlor, Methoxy oder Ethoxy;

Die Benzoylhalogenide der Formel IIIα' (mit L^{1'} = Cl, Br,) können auf an sich bekannte Art und Weise durch Umsetzung der Benzoesäuren der Formel IIIβ mit Halogenierungsreagentien wie Thionylchlorid, Thionylbromid, Phosgen, Diphosgen, Triphosgen, Oxalylchlorid, Oxalylbromid hergestellt werden.

Die Benzoesäuren der Formel IIIβ können in bekannter Weise durch saure oder basische Hydrolyse aus den entsprechenden Estern der Formel IIIγ (L³ = C₁-C₆-Alkoxy) hergestellt werden.

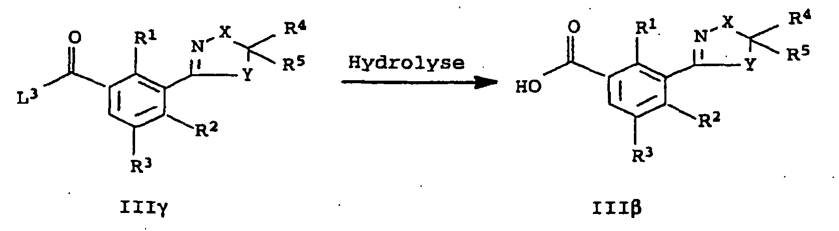

Ebenso können die Benzoesäuren der Formel IIIß durch Umsetzung von entsprechenden Brom- oder Iod-substituierten Verbindungen der Formel V, in Gegenwart eines Palladium-, Nickel-, Cobalt- oder Rhodium-Übergangsmetallkatalysators und einer Base mit Kohlenmonoxid und Wasser unter erhöhtem Druck erhalten werden.

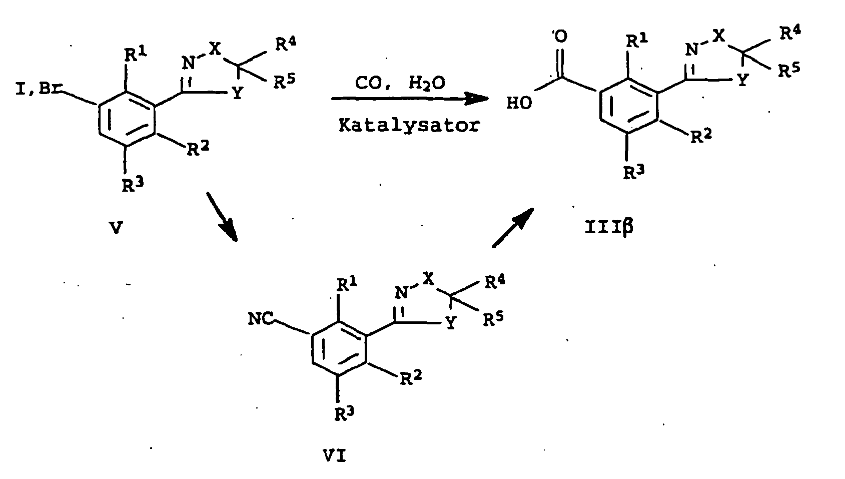

Weiterhin ist es möglich durch Rosenmund-von Braun-Reaktion Verbindungen der Formel V in die entsprechenden Nitrile der Formel VI zu überführen (vgl. z.B. Org. Synth. Bd III, 212 (1955)) und diese durch nachfolgende verseifung in die Verbindungen der Formel IIIβ umzuwandeln.

Die Ester der Formel IIIγ können durch Umsetzung von Arylhalogenverbindungen oder Arylsulfonaten der Formel VII, wobei L⁴ für eine Ausgangsgruppe wie Brom, Iod, Triflat, Fluorsulfonyloxy etc. steht, mit Heterocyclyl-Stannaten (Stille-Kupplungen), Heterocyclyl-Borverbindungen (Suzuki-Kupplungen) oder Heterocyclyl-Zinkverbindungen (Negishi-Reaktion) VIII, wobei M entsprechend für Sn(C₁-C₄-Alkyl)₃, B(OH)₂, ZnHal (mit Hal = Chlor, Brom) etc. steht, auf an sich bekannte Art und Weise (vgl. z.B. Tetrahedron Lett. 27, 5269 (1986) in Gegenwart eines Palladium- oder Nickel-Übergangsmetallkatalysator und gegebenenfalls einer Base erhalten werden.

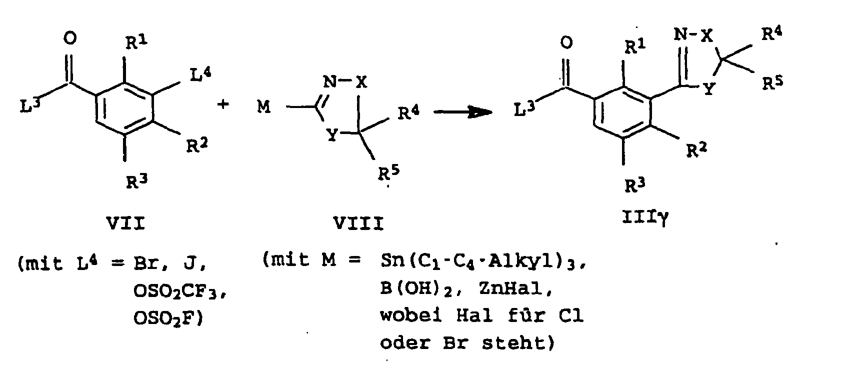

Ebenso ist es möglich, Ester der Formel IIIγ durch Aufbau des in 3-Position gebundenen Heterocycluses zu erhalten.

Beispielsweise können aus Amidoximen der Formel IX durch Kondensation mit Aldehyden oder Ketonen 1,2,4-Oxadiazolin-3-yl-Derivate (IIIγ mit X=O. Y=NH) hergestellt werden (vgl. z.B. Arch. Phar-326, 383-389 (1993)).

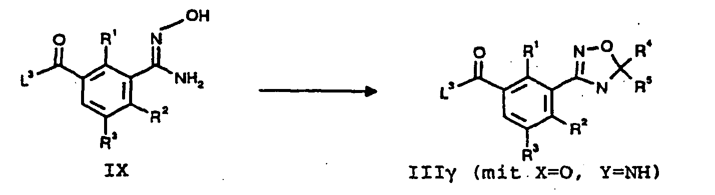

Die Thioamide der Formel X sind geeignete Vorprodukte für 2-Thiazolinyl-Derivate I (mit X=CR¹⁰R¹¹, Y=S) (vgl. z.B. Tetrahedron 42, 1449-1460 (1986)).

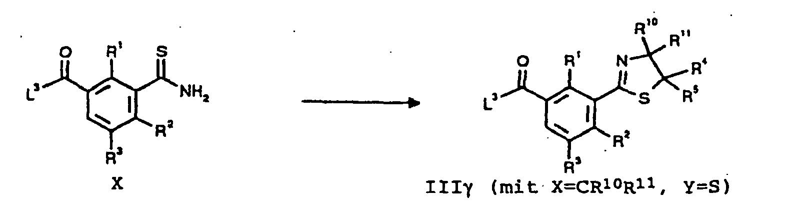

Aus den Carbonsäuren der Formel XI sind 2-Oxazolinyl-, 2-Thiazolinyl- und 2-Imidazolinyl-Derivate (IIIγ mit X=CR¹⁰R¹¹, Y=O bzw. Y=S bzw. Y=NH) zugänglich (vgl. z.B. Tetrahedron Let. 22, 4471-4474 (1981)).

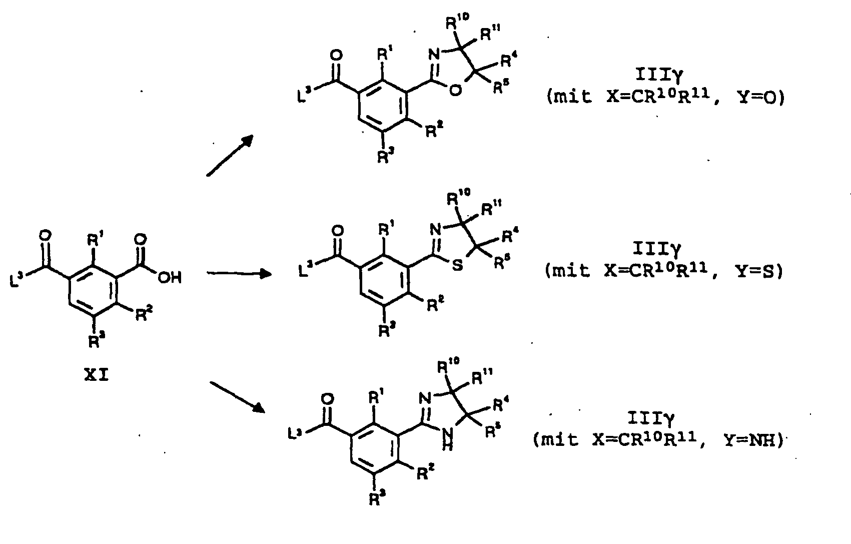

Nach literaturbekannten Verfahren können aus Carbonsäurehalogeniden der Formel XII, wobei Hal für Halogen steht, insbesondere aus Carbonsäurechloriden, 1,3-Thiazol-5(4H)-thion-2-yl- (vgl. z.B. Helv. Chim. Acta, 69, 374-388 (1986)) und S-Oxo-2-imidazolin-2-yl-Derivate (vgl. z.B. Heterocycles 29, 1185-1189 (1989)) (III mit X=CR¹⁰R¹¹, Y=S bzw. Y=NH) hergestellt werden.

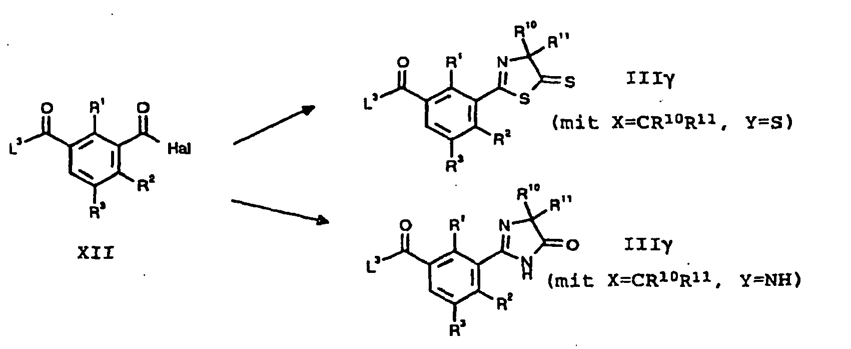

Die Umwandlung von Oximen der Formel XIII in 4,5-Dihydro-isoxazol-3-yl-Derivate (mit IIIγ mit X=O, Y=CR¹³R¹⁴) kann in an sich bekannter Weise über die Zwischenstufe der Hydroxamsäurechloride XIV erfolgen. Aus letzteren werden in situ Nitriloxide erzeugt, die mit Alkenen zu den gewünschten Produkten abreagieren (vgl. z.B. Chem. Ber. 106, 3258-3274 (1973)). 1,3-Dipolare Cycloadditionen von Chlorsulfonylisocyanat an Nitriloxide liefern 1,2,4-Oxadiazolin-5-on-3-yl-Derivate (IIIγ mit X=O, Y=NH) (vgl. z.B. Heterocycles 27, 683-685 (1988)).

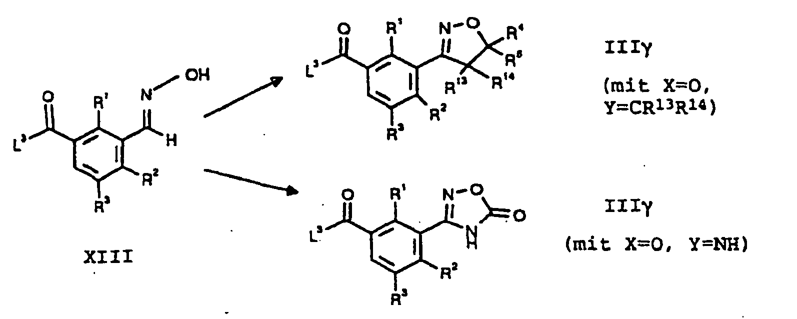

Die Aldehyde der Formel XIV können über die Zwischenstufe der Semicarbazone in 2,4-Dihydro-1,2,4-triazol-3-on-5-yl-Derivate (IIIγ mit X=NR⁹, X=NR¹²) umgewandelt werden (vgl.z.B. J. Heterocyclic Chem., 23, 881- 883 (1986)).

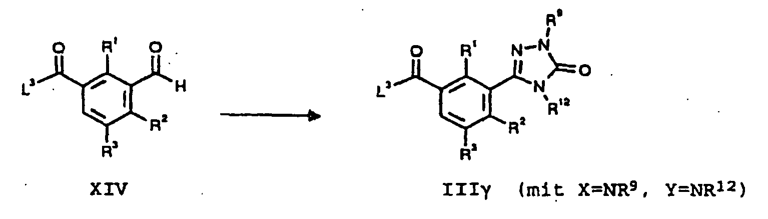

2-Imidazolinyl-Derivate (IIIγ mit X=CR¹⁰R¹¹, Y=NH) sind auch aus Benzonitrilen der Formel XV nach bekannten Methoden (vgl. z.B. J. Org. Chem. 52, 1017-1021 (1987)) herzustellen.

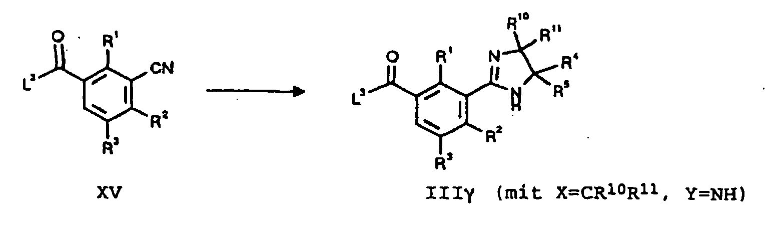

Mittels 1,3-dipolarer Cycloaddition von Diazoalkanen bzw. Nitriliminen mit Arylalkenen der Formel XVI können 3-Pyrazolinyl-Derivate (IIIγ mit X=NH, Y=CHR¹³) dargestellt werden.

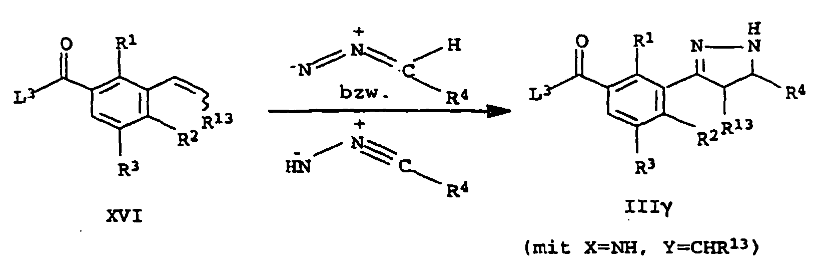

Die als Ausgangsverbindungen verwendeten Brom- oder Iod-substituierten Verbindungen der Formel V können in Analogie zu literaturbekannten Methode, z.B. durch Sandmeyer-Reaktion aus entsprechenden Anilinen erhalten werden, die ihrerseits durch Reduktion geeigneter Nitroverbindungen synthetisiert werden. Die Brom-substituierten Verbindungen der Formel V können außerdem durch direkte Bromierung geeigneter Edukte erhalten werden (vgl. Monatsh. Chem. 99, 815-822 (1968)).

Die Nitrile der Formel VI können wie oben beschrieben erhalten werden. Ebenso ist es möglich, diese aus entsprechenden Anilinen mittels Sandmeyer-Reaktion darzustellen.

Die Ausgangsverbindungen der Formel VII sind bekannt (vgl. z.B. Coll. Czech. Chem. Commun. 40, 3009-3019 (1975)) oder können leicht durch geeignete Kombination bekannter Synthesen hergestellt werden.

Beispielsweise können die Sulfonate VII (L⁴ = OSO₂CF₃, OSO₂F) aus den entsprechenden Phenolen, die ihrerseits bekannt sind (vgl. z.B. EP-A 195 247) oder nach bekannten Methoden hergestellt werden können, erhalten werden (vgl. z.B. Synthesis 1993, 735-762).

Die Halogenverbindungen VII (L⁴ = C1, Br oder I) können beispielsweise durch Sandmeyer-Reaktion aus entsprechenden Anilinen der Formel XIX erhalten werden.

Die Amidoxime der Formel IX, die Thioamide der Formel X und die Carbonsäuren der Formel XI können auf an sich bekannte Art und Weise aus den Nitrilen der Formel XV dargestellt werden.

Weiterhin ist es möglich, die Carbonsäuren der Formel XI aus den Aldehyden der Formel XIV nach bekannten Verfahren herzustellen (vgl. z.B. J. March, Advanced Organic Chemistry, 3. Auflage, S. 629 ff, Wiley-Interscience Publication (1985)).

Die Carbonsäurehalogenide der Formel XII können in Analogie zu Standardverfahren aus den entsprechenden Carbonsäuren der Formel XI erhalten werden.

Die Oxime der Formel XIII erhält man vorteilhaft dadurch, daß man in an sich bekannter Weise Aldehyde der Formel XIV mit Hydroxylamin umsetzt (vgl. z.B. J. March. Advanced Organic Chemistry, 3. Aufl., S. 805-806, Wiley-Interscience Publication (1985)).

Die Aldehyde der Formel XIV sind bekannt oder in Analogie zu bekannten Verfahren darstellbar. So können sie aus Methylverbindungen der Formel XVII durch Bromierung, beispielsweise mit N-Bromsuccinimid oder 1,3-Dibrom-5,5-dimethylhydantoin, und anschließende Oxidation dargestellt werden (vgl. Synth. Commun. 22, 1967 - 1971 (1992)).

Die Umwandlung der Oxime der Formel XIII in Nitrile der Formel XV kann ebenfalls nach an sich bekannten Verfahren erfolgen (vgl. z.B. J. March, Advanced Organic Chemistry, 3. Aufl., S. 931-932, Wiley-Interscience Publication (1985))**.**

Ausgehend von den Halogenverbindungen oder Sulfonaten der Formel VII (L⁴ = Br, Cl, OSO₂CF₃, OSO₂F) lassen sich u.a. durch Heck-Reaktion mit Olefinen in Gegenwart eines Palladiumkatalysators Arylalkene der Formel XVI darstellen (vgl. z.B. Heck, Palladium Reagents in Organic Synthesis, Academic Press, London 1985; Synthesis 1993, 735 - 762).

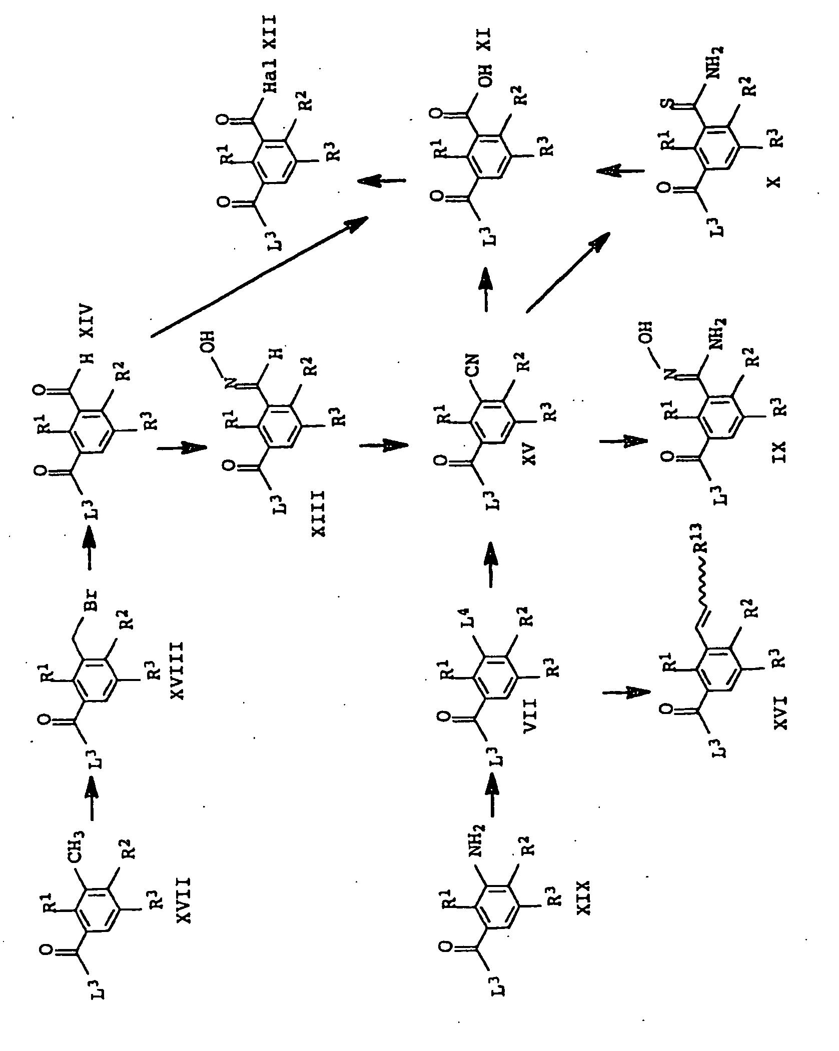

### Herstellungsbeispiele:

### 4-[2-Chlor-3-(4,5-dihydroisoxazol-3-yl)-4-methylsulfonyl-benzoyl]-5-hydroxy-1-methyl-1H-pyrazol (nicht erfindungsgemäß)

Zu einer Lösung von 12,74 g (0,13 mol) 5-Hydroxy-1-methyl-pyrazol und 300 ml wasserfreiem Dioxan wurden unter Schutzgasatmosphäre bei Raumtemperatur gleichzeitig 43,60 g (0,13 mol) 2-Chlor-3-(4,5-dihydroisoxazol-3-yl)-4-methylsulfonyl-benzoyl-chlorid in 375 ml wasserfreiem Dioxan und 13,56 g (0,134 mol) Triethylamin in 375 ml wasserfreiem Dioxan getropft. Nach 2. Stunden Rühren bei Raumtemperatur wurde das Reaktionsgemisch über Kieselgel abfiltriert und mit Dioxan nachgewaschen. Das Eluat wurde am Vakuum auf ca. 500 ml eingeengt und mit 17,94 (0,13 mol) getrocknetem, fein gepulvertem Kaliumcarbonat versetzt. Nach 6 Stunden Erhitzen unter Rückfluß wurde das Lösungsmittel am Vakuum abdestilliert und der Rückstand in ca. 700 ml Wasser aufgenommen. Unlösliche Bestandteile wurden abfiltriert und der pH-Wert des Filtrats durch langsame Zugabe von 10 %iger Salzsäure auf pH = 2 - 3 eingestellt. Der sich bildende Niederschlag wurde abgesaugt. Man erhielt 46,16 g (92 % d. Th.) 4-[2-Chlor-3-(4,5-dihydroisoxazol-3-yl)-4-methylsulfonyl-benzoyl]-5-hydroxy-1-methyl-1H-pyrazol.
(Fp. > 250°C)

In Tabelle 3 sind noch weitere 3-Heterocyclyl-substituierte Benzoylderivate der Formel I aufgerührt, die in analoger Weise hergestellt wurden oder herstellbar sind (falls die Endprodukte beim Ansäuern mit 10 %iger Salzsäure nicht ausgefallen sind, wurden sie mit Essigsäureethylester oder Dichlormethan extrahiert; anschließend wurde die organische Phase getrocknet und am Vakuum eingeengt):

**Tabelle 3:**

| | | | | | | | | | | | |
|---|---|---|---|---|---|---|---|---|---|---|---|
| 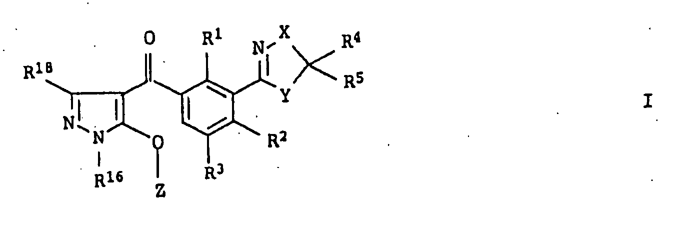 | | | | | | | | | | | |

| Nr. | R¹ | R² | R³ | X | R⁴ | R⁵ | Y | R¹⁶ | Z | R¹⁸ | physikalische Daten Fp. [°C]; ¹H-NMR [δ in ppm] |
|---|---|---|---|---|---|---|---|---|---|---|---|
| 3.86 | CH₃ | SO₂CH₃ | H | O | H | H | CH₂ | CH₃ | H | H | 205-210 |
| 3.90 | CH₃ | SO₂CH₃ | H | O | H | H | CH₂ | C₂H₅ | H | H | 174-180 |
| 3.111 | SO₂CH₃ | SO₂CH₃ | H | O | H | H | CH₂ | CH₃ | H | H | Öl |
| 3.112 | SO₂CH₃ | SO₂CH₃ | H | O | H | H | CH₂ | C₂H₅ | H | H | Öl |

Nachfolgend sind die Synthesen einiger Ausgangsstoffe aufgeführt:

### 2-Chlor-3-(4,5-dihydroisoxazol-3-yl)-4-methylsulfonyl-benzoylchlorid (nicht erfindungsgemäß)

### Stufe a) 2-Chlor-3-methyl-4-methylthio-acetophenon

Zu einer Suspension von 286 g (2,14 mol) Aluminiumtrichlorid in 420 ml 1,2-Dichlorethan wurde bei 15-20°C eine Lösung von 157 g (2 mol) Acetylchlorid in 420 mol 1,2-Dichlorethan getropft. Anschließend wurde eine Lösung von 346 g (2 mol) 2-Chlor-6-methylthio-toluol in 1 1 1,2-Dichlorethan zugetropft. Nach 12 Stunden Rühren wurde das Reaktionsgemisch in eine Mischung aus 3 1 Eis und 1 1 konz. HCl gegossen. Es wurde mit Methylenchlorid extrahiert, die organische Phase mit Wasser gewaschen, mit Natriumsulfat getrocknet und eingeengt. Der Rückstand wurde im Vakuum destilliert. Man erhielt 256 g (60 % d.Th.) 2-Chlor-3-methyl-4-methyl-thio-acetophenon.
(Fp.: 46°C)

### Stufe b) 2-Chlor-3-methyl-4-methylsulfonyl-acetophenon

163,0 g (0,76 mol) 2-Chlor-3-methyl-4-methylthio-acetophenon wurden in 1,5 1 Eisessig gelöst, mit 18,6 g Natriumwolframat versetzt und unter Kühlung 173,3 g 30 %ige Wasserstoffperoxidlösung zugetropft. Es wurde 2 Tage nachgerührt und anschließend mit Wasser verdünnt. Der ausgefallene Feststoff wurde abgesaugt, mit Wasser gewaschen und getrocknet. Man erhielt 164,0 g (88 % d.Th.) 2-Chlor-3-methyl-4-methylsulfonyl-acetophenon.
(Fp.: 110-111°C)

### Stufe c) 2-Chlor-3-methyl-4-methylsulfonyl-benzoesäure

82 g (0,33 mol) 2-Chlor-3-methyl-4-methylsulfonyl-acetophenon wurden in 700 ml Dioxan gelöst und bei Raumtemperatur mit 1 1 einer 12,5 %igen Natriumhypochloritlösung versetzt. Anschließend wurde 1 Stunde bei 80°C nachgerührt. Nach dem Abkühlen bildeten sich zwei Phasen, von denen die untere mit Wasser verdünnt und schwach angesäuert wurde. Der ausgefallene Feststoff wurde abgesaugt, mit Wasser nachgewaschen und getrocknet. Man erhielt 60 g (73 % d.Th-) 2-Chlor-3-methyl-4-methylsulfonyl-benzoesäure.
(Fp.: 230-231°C)

### Stufe d) 2-Chlor-3-methyl-4-methylsulfonyl-benzoesäuremethylester

100 g (0,4 mol) 2-Chlor-3-methyl-4-methylsulfonyl-benzoesäure wurden in 1 1 Methanol gelöst und bei Rückflußtemperatur 5 Stunden mit Chlorwasserstoff begast. Anschließend wurde eingeengt. Man erhielt 88.5 g (84 % d.Th.) 2-Chlor-3-methyl-4-methylsulfonyl-benzoesäuremethylester.
(Fp.: 107-108°C)

### Stufe e) 3-Brommethyl-2-chlor-4-methylsulfonyl-benzoesäuremethylester

82 g (0,31 mol) 2-Chlor-3-methyl-4-methylsulfonyl-benzoesäuremethylester werden in 2 1 Tetrachlormethan gelöst und unter Belichtung portionsweise mit 56 g (0,31 mol) N-Bromsuccinimid versetzt. Das Reaktionsgemisch wurde filtriert, das Filtrat eingeengt und der Rückstand in 200 ml Methyl-tert.-butylether aufgenommen. Die Lösung wurde mit Petrolether versetzt, der ausgefallene Feststoff abgesaugt und getrocknet. Man erhielt 74,5 g (70 % d.Th.) 3-Brommethyl-2-chlor-4-methylsulfonyl-benzoesäuremethylester.
(Fp.: 74-75°C)

### Stufe f) 2-Chlor-3-formyl-4-methylsulfonyl-benzoesäuremethylester

Eine Lösung von 41,0 g (0,12 mol) 3-Brommethyl-2-chlor-4-methylsulfonyl-benzoesäuremethylester in 250 ml Acetonitril wurde mit 42,1 g (0,36 mol) N-Methylmorpholin-N-oxid versetzt. Der Ansatz wurde 12 Stunden bei Raumtemperatur gerührt, anschließend eingeengt und der Rückstand in Essigsäureethylester aufgenommen. Die Lösung wurde mit Wasser extrahiert, mit Natriumsulfat getrocknet und eingeengt. Man erhielt 31,2 g (94 % d.Th.)
2-Chlor-3-formyl-4-methylsulfonyl-benzoesäuremethylester
(Fp.: 98-105°C)

### Stufe g) 2-Chlor-3-hydroxyiminomethyl-4-methylsulfonyl-benzoesäure

15,00 g (54 mmol) 2-Chlor-3-formyl-4-methylsulfonyl-benzoesäuremethylester und 4,20 g (60 mmol) Hydroxylaminhydrochlorid wurden in 300 ml Methanol aufgenommen und eine Lösung von 3,18 g (30 mmol) Natriumcarbonat in 80 ml Wasser zugetropft. Nach 12 Stunden Rühren bei Raumtemperatur wurde das Methanol abdestilliert, der Rückstand mit Wasser verdünnt und mit Diethylether extrahiert. Nach Trocknen der organischen Phase wurde das Lösungsmittel entfernt. Man erhielt 14,40 g (91 % d.Th.)
2-Chlor-3-hydroxyiminomethyl-4-methylsulfonyl-benzoesäuremethylester.
(Fp.: 126-128°C) .

### Stufe h) 2-Chlor-3-(4,5-dihydroisoxazol-3-yl)-4-methylsulfonyl-benzoesäuremethylester

In eine Lösung von 158,0 g (0,54 mol) 2-Chlor-3-hydroxyiminomethyl-4-methylsulfonyl-benzoesäuremethylester und 1 1 Dichlormethan wurde bei 15-20°C 30 Minuten lang Ethylen eingeleitet. Nach Zugabe von 1,6 g Natriumacetat wurden 454 ml Natriumhydrochlorit-Lösung bei 10°C unter gleichzeitiger Ethylen-Einleitung zugetropft. Anschlieβend wurde für weitere 15 Minuten Ethylen bei 10°C eingeleitet. Nach 12 Stunden Rühren wurden die Phasen getrennt, die organische Phase mit Wasser gewaschen, getrocknet und eingeengt. Man erhielt 156,5 g (90 % d.Th.) 2-Chlor-(4,5-dihydroisoxazol-3-yl)-4-methylsulfonyl-benzoesäuremethylester.
(¹H-NMR (ö in ppm): 3,24 (s); 3,42 (t); 3,99 (s); 4,60 (t) 7,96 (d); 8,10 (d)).

### Stufe i) 2-Chlor-3-(4,5-dihydroisoxazol-3-yl)-4-methylsulfonylbenzoesäure

Zu einem Gemisch von 170,0 g (0,54 mol) 2-Chlor-3-(4,5-dihydroisoxazol-3-yl) -4-methylsulfonyl-benzoesäuremethylester und 1 1 Methanol wurde bei 40-45°C langsam eine Lösung von 32,8 g Natriumhydroxid gelöst in 330 ml Methanol getropft. Die Suspension wurde 5 Stunden bei 50°C gerührt. Nach Abdestillieren des Lösungsmittels nahm man den Rückstand in 1,5 1 Wasser auf und extrahierte die wäßrige Phase dreimal mit Essigsäureethylester. Die wäßrige Phase wurde mit Salzsäure angesäuert und dreimal mit Essigsäureethylester extrahiert. Die vereinigten organischen Phasen wurden anschließend mit Wasser neutral gewaschen, getrocknet und eingeengt. Man erhielt 148,8 g (91 % d.Th.) 2-Chlor-3-(4,5-dihydroisoxazol-3-yl)-4-methylsulfonyl-benzoesäure.
(¹H-NMR (δ in ppm): 3,26 (s); 3,45 (t); 4,63 (t); 8,15 (s); 8,53 (s, br)).

### Stufe j) 2-Chlor-3-(4,5-dihydroisoxazol-3-yl)-4-methylsulfonylbenzoylchlorid

Zu einer Lösung von 139,0 g 2-Chlor-3-(4,5-dihydroisoxazol-3-yl)-4-methylsulfonyl-benzoesäure, 1 ml Dimethylformamid und 1 1 trockenem Toluol wurden bei 50°C 74,8 g (0,63 mol) Thionylchlorid in 50 ml trockenem Toluol getropft. Nach 6 Stunden Erhitzen auf 110°C wurde das Lösungsmittel abdestilliert. Man erhielt 2-Chlor-3-(4,5-dihydroisoxazol-3-yl)-4-methylsulfonyl-benzoylchlorid in quantitativer Ausbeute.
(¹H-NMR (δ in ppm): 3,25 (s); 3,46 (t): 4,62 (t); 8,21 (dd)).

### 2-Chlor-3-(5-methyl-4,5-dihydroisoxazol-3-yl)-4-methylsulfonyl-benzoylchlorid (nicht erfindungsgemäß)

### Stufe a) 2-Chlor-3-(5-methyl-4,5-dihydroisoxazol-3-yl)-4-methylsulfonyl-benzoesäuremethylester

In eine Lösung von 15,0 g (52 mmol) 2-Chlor-3-hydroxyiminomethyl-4-methylsulfonyl-benzoesäuremethylester und 200 ml Dichlormethan wurde bei Raumtemperatur 30 Minuten lang Propen eingeleitet. Nach Zugabe von 1,6 g Natriumacetat wurden 42,8 ml Natriumhydrochlorit-Lösung bei Raumtemperatur unter gleichzeitiger Propen-Einleitung zugetropft. Anschließend wurde für weitere 15 Minuten Propen bei Raumtemperatur eingeleitet. Nach 3 Stunden Erhitzen unter Rückfluß wurde 12 Stunden bei Raumtemperatur gerührt, nochmals 5 Stunden unter Rückfluß Propen eingeleitet und wiederum 12 Stunden bei Raumtemperatur gerührt. Nach Trennung der Phasen wurde die organische Phase mit Wasser gewaschen, getrocknet und eingeengt. Man erhielt 15,5 g (89 % d.Th.) 2-Chlor-(5-methyl-4,5-dihydroisoxazol-3-yl)-4-methylsulfonyl-benzoesäuremethylester.
(Fp.: 130-135°C).

### Stufe b) 2-Chlor-3-(5-methyl-4,5-dihydroisoxazol-3-yl)-4-methylsulfonyl-benzoesäure :

Zu einem Gemisch von 15,00 g (45 mmol) 2-Chlor-3-(5-methyl-4,5-dihydroisoxazol-3-yl)-4-methylsulfonyl-benzoesäuremethylester und 200 ml Methanol wurde langsam eine Lösung von 3,52 g (88 mmol) Natriumhydroxid gelöst in 100 ml Methanol getropft. Die Suspension wurde 48 Stunden bei Raumtemperatur gerührt. Nach Abdestillieren des Lösungsmittels nahm man den Rückstand in Wasser auf und wusch die wäßrige Phase dreimal mit Essigsäureethylester. Die wäßrige Phase wurde mit Salzsäure angesäuert und dreimal mit Essigsäureethylester extrahiert. Die vereinigten organischen Phasen wurden anschließend mit Wasser neutral gewaschen, getrocknet und eingeengt. Man erhielt 13,20 g (92 % d.Th.) 2-Chlor-3-(5-methyl-4,5-dihydroisoxazol-3-yl)-4-methylsulfonyl-benzoesäure.
(Fp.: 173-178°C).

### Stufe c) 2-Chlor-3-(5-methyl-4,5-dihydroisoxazol-3-yl)-4-methylsulfonyl-benzoylchlorid

Zu einer Lösung von 13,0 g (41 mmol) 2-Chlor-3-(5-methyl-4,5-dihydroisoxazol-3-yl)-4-methylsulfonylbenzoesäure, 1 ml Dimethylformamid und 250 ml trockenem Toluol wurden bei Raumtemperatur 5,7 g (51 mmol) Thionylchlorid getropft. Anschließend wurde bis zur vollständigen Umsetzung unter Rückfluß erhitzt. Nach Abkühlen wurde das Lösungsmittel abdestilliert. Man erhielt 14,2 g 2-Chlor-3-(5-methyl-4,5-dihydroisoxazol-3-yl)-4-methylbenzoyl-chlorid in quantitativer Ausbeute.

### 2-Chlor-3-(1'-chlor-2',2'-dimethylethylaminocarbonyl)-4-methylsulfonyl-benzoylchlorid (nicht erfindungsgemäß)

### Stufe a) 2-Chlor-3-hydroxycarbonyl-4-methylsulfonyl-benzoesäuremethylester

Zu einer Lösung von 115,3 g (0,42 mol) 2-Chlor-3-formyl-4-methylsulfonyl-benzoesäuremethylester und 2000 ml Acetonitril wurden bei 5°C nacheinander 13,8 g (0,11 mol) Natriumhydrogenphosphatmonohydrat in 170 ml Wasser, 49,3 g (0,43 mol) 30 %ige Wasserstoffperoxidlösung und 66,2 g (0,59 mol) 80 %ige wäßrige Natriumchloritlösung gegeben. Die Reaktionslösung wurde anschließend 1 Stunde bei 5°C und 12 Stunden bei Raumtemperatur gerührt. Dann wurde mit 10 %iger Salzsäure auf pH = 1 eingestellt und 1500 ml wäßrige 40 %ige Natriumhydrogensulfit-Lösung zugegeben. Nach 1 Stunde Rühren bei Raumtemperatur wurde die wäßrige Phase dreimal mit Essigsäureethylester extrahiert. Die vereinigten organischen Phasen wurden mit Natriumhydrogensulfit-Lösung gewaschen und getrocknet. Nach Abdestillation des Lösungsmittels erhielt man 102,0 g 2-Chlor-3-hydroxycarbonyl-4-methylsulfonyl-benzoesäuremethylester.
(¹H-NMR (δ in ppm) : 3,34 (s) ; 3, 93 (s) ; 8,08 (s) ; 14, 50 (s, br.).)

### Stufe b) 2-Chlor-3-chlorcarbonyl-4-methylsulfonyl-benzoesäuremethylester

Zu einer Lösung von 6,0 g (0,021 mol) 2-Chlor-3-hydroxy-carbonyl-4-methylsulfonyl-benzoesäuremethylester und 50 ml trockenem Toluol wurden 2 Tropfen Dimethylformamid und 11,9 g (0,1 mol) Thionylchlorid gegeben. Die Lösung wurde 4 Stunden unter Rückfluß erhitzt. Nach Entfernen des Lösungsmittels am Vakuum erhielt man 6,2 g 2-Chlor-3-chlorcarbonyl-4-methylsulfonyl-benzoesäuremethylester.
(¹H-NMR (δ in ppm) : 3, 21 (s) ; 4,02 (s) ; 8,02 (d) ; 8, 07 (d).)

### Stufe c) 2-Chlor-3-(1'-hydroxy-2',2'-dimethylethylaminocarbonyl)-4-methylsulfonyl-benzoesäuremethylester

Zu einer Lösung von 4,54 g (50 mmol) 2,2-Dimethylethanolamin in 40 ml Dichlormethan wurde bei 0-5°C eine Lösung von 7,80 g (25 mmol) 2-Chlor-3-chlorcarbonyl-4-methylsulfonyl-benzoesäuremethylester getropft. Nach 6 Stunden Rühren bei Raumtemperatur wurde die Reaktionslösung dreimal mit Wasser extrahiert, getrocknet und eingeengt. Man erhielt 8,20 g (80 % d.Th.) 2-Chlor-3-(1'-hydroxy-2',2'-dimethylethylaminocarbonyl)-4-methylsulfonyl-benzoesäuremethylester.
(Fp.: 70-72°C).

### Stufe d) 2-Chlor-3-(1'-chlor-2',2'-dimethylethylaminocarbonyl)-4-methylsulfonyl-benzoesäuremethylester

Ein Gemisch aus 6,9 g (20 mmol) 2-Chlor-3-(1'-hydroxy-2',2'-dimethylethylaminocarbonyl)-4-methylsulfonyl-benzoesäuremethylester und 5 ml Thionylchlorid wurde 6 Stunden bei Raumtemperatur gerührt. Die Lösung wurde mit 50 ml Dichlormethan verdünnt und anschließend eingeengt. Der Rückstand wurde in 20 ml Dichlormethan gelöst. Durch Zugabe von Cyclohexan bildete sich ein kristalliner Niederschlag, der abgesaugt und getrocknet wurde. Man erhielt 6,4 g (88 % d.Th.) 2-Chlor-3-(1'-chlor-2',2'-dimethylethylaminocarbonyl)-4-methylsulfonyl-benzoesäuremethylester.

### Stufe e) 2-Chlor-3-(4',4'-dimethyl-4',5'-dihydroxazol-2-yl)-4-methylsulfonyl-benzoesäure

Eine Lösung von 5,82 g (15 mmol) 2-Chlor-3-(1'-chlor-2',2'-dimethylethylaminocarbonyl)-4-methylsulfonyl-benzoesäuremethylester und 0,81 g (20 mmol) Natriumhydroxid in 80 ml Methanol rührte 8 Stunden bei Raumtemperatur. Nach Abdestillieren des Lösungsmittels wurde der Rückstand in Wasser aufgenommen und dreimal mit Essigsäureethylester gewaschen. Die wäßrige Phase wurde mit Salzsäure angesäuert und dreimal mit Essigsäureethylester extrahiert. Nach dem Trocknen der organischen Phase entfernte man das Lösungsmittel am Vakuum. Man erhielt 3,10 g (56 % d.Th.) 2-Chlor-3-(4',4'-dimethyl-4',5'-dihydrooxazol-2-yl)-4-methylsulfonyl-benzoesäure.
(¹H-NMR (δ in ppm): 1,34 (s); 3,40 (s): 4,13 (s); 8,07 (s); 13,95 (s, br)).

### Stufe f) 2-Chlor-3-(1'-chlor-2',2'-dimethylethylaminocarbonyl)-4-methylsulfonyl-benzoylchlorid

Eine Lösung von 3,00 g (9 mmol) 2-Chlor-3-(4',4'-dimethyl-4',5'-dihydrooxazol-2-yl)-4-methylsulfonyl-benzoesäure, 1,43 g Thionylchlorid und 1 Tropfen Dimethylformamid in 80 ml trockenem Toluol wurde 3 Stunden unter Rückfluß erhitzt. Nach dem Abkühlen wurde das Lösungsmittel am Vakuum abdestilliert. Man erhielt 3,43 g (86 % d.Th.) 2-Chlor-3-(1'-chlor-2',2'-dimethylethylaminocarbonyl)-4-methylsulfonyl-benzoylchlorid.

### 2-Chlor-3-(1,3,4-oxathiazolin-2-on-5-yl)-4-methylsulfonyl-benzoesäuremethylester (nicht erfindungsgemäß)

### Stufe a) 3-Aminocarbonyl-2-chlor-4-methylsulfonyl-benzoesäuremethylester .

In eine Lösung von 15,0 g (48 mmol) 2-Chlor-3-chlorcarbonyl-4-methylsulfonyl-benzoesäuremethylester und 300 ml trockenem Dioxan wurde 2 Stunden lang Ammoniak geleitet. Der gebildete Niederschlag wurde abgesaugt und das Filtrat eingeengt. Man erhielt 15,2 g 3-Aminocarbonyl-2-chlor-4-methylsulfonyl-benzoesäuremethylester in quantitativer Ausbeute.

### Stufe b) 2-Chlor-3-(1,3,4-oxathiazolin-2-on-5-yl)-4-methylsulfonyl-benzoesäuremethylester

Zu einer Lösung von 4,37 g (15 mmol) 3-Aminocarbonyl-2-chlor-4-methylsulfonyl-benzoesäuremethylester in 150 ml trockenem Toluol wurden 9,80 g (75 mmol) Chlorcarbonylsulfenylchlorid getropft. Nach 48 Stunden Rühren unter Rückfluß wird das Lösungsmittel am Vakuum entfernt und der Rückstand am Kieselgel chromatographiert (Eluent: Essigsäureethylester/Cyclohexan = 1/1). Man erhielt 3,70 g (70 % d.Th.) 2-Chlor-3-(1,3,4-oxathiazolin-2-on-5-yl)- 4-methylsulfonyl-benzoesäuremethylester.

### 2-Chlor-4-methylsulfonyl-3-(4,5-dihydrooxazol-2-yl)-benzoesäuremethylester (nicht erfindungsgemäß)

Zu 26,6 g (0,13 mol) 1-Amino-2-bromethan-Hydrobromid in 500 ml Toluol wurden bei Raumtemperatur 41,8 g (0,41 mol) Triethylamin und anschließend 31,1 g (0,10 mol) 2-chlor-3-chlorcarbonyl-4-methylsulfonylbenzoesäuremethylester in 150 ml Toluol getropft. Nach 5 Stunden Erhitzen unter Rückfluß und 12 Stunden Rühren bei Raumtemperatur wurden nochmals 5,0 g (0,02 mol) 1-Amino-2-bromethan-Hydrobromid zugegeben und 7,5 Stunden unter Rückfluß erhitzt. Nach Abkühlen wurde das Reaktionsgemisch mit Essigsäureethylester verdünnt, mit Wasser gewaschen, getrocknet und eingeengt. Der Rückstand wurde dann aus Methyl-tert.-butylether/Essigsäureethylester umkristallisiert. Man erhielt 14,5 g (46 % d.Th.) 2-Chlor-4-methylsulfonyl-3-(4,5-dihydroxazol-2-yl)-benzoesäuremethylester.

### 2-Chlor-3-(5-methoxy-5-methyl-4,5-dihydroisoxazol-3-yl)-4-methylsulfonyl-benzoesäure (nicht erfindungsgemäß)

### Stufe a) 2-Chlor-3- (5-methoxy-5-methyl-4,5-dihydroisoxazol-3-yl) - 4-methylsulfonyl-benzoesäuremethylester

Zu 10,0 g (34 mmol) 2-Chlor-3-(hyclroxyiminomethyl)-4-methylsulfonyl-benzoesäuremethylester in 200 ml Methylenchlorid wurden nacheinander 7,3 g (102 mmol) 2-Methoxy-1-propen, 28 ml Natriumhypochloritlösung (12,5 %ig) und eine Spatelspitze Natriumacetat zugegeben. Nach 12 Stunden Rühren bei Raumtemperatur wurde das Lösungsmittel entfernt, der Rückstand in Essigsäureethylester aufgenommen, mit Wasser gewaschen, getrocknet und eingeengt. Der Rückstand wurde an Kieselgel (Eluent: Cyclohexan : Essigsäureethylester = 3:2) chromatographiert. Man erhielt 5,8 g (47 % d.Th.) 2-Chlor-3-(5-methoxy-5-methyl-4,5-dihydroisoxazol-3-yl)-4-methylsulfonyl-benzoesäuremethylester.
(Fp.: 100-105°C)

### Stufe b) 2-Chlor-3-(5-methoxy-5-methyl-4,5-dihydroisoxazol-3-yl)-4-methylsulfonyl-benzoesäure

Zu 5,0 g (37,5 mmol) Lithiumiodid in 200 ml Pyridin wurden bei Rückflußtemperatur 5,5 g (15,0 mmol) 2-Chlor-3-(5-methoxy-5-methyl-4,5-dihydroisoxazol-3-yl)-4-methylsulfonyl-benzoesäuremethylester in 100 ml Pyridin getropft. Nach 4 Stunden Rühren bei dieser Temperatur wurde abgefühlt, das Lösungsmittel abdestilliert, der Rückstand in Toluol aufgenommen und wiederum eingeengt. Anschließend wurde mit Wasser versetzt, mit Methylenchlorid gewaschen und mit Salzsäure ein pH-Wert von 1 eingestellt. Nach Extraktion der wäßrigen Phase mit Methylenchlorid wurde die resultierende organische Phase getrocknet und eingeengt. Man erhielt 4,7 g (90 % d.Th.) 2-Chlor-(5-methoxy-5-methyl-4,5-dihydroisoxazol-3-yl)-4-methylsulfonyl-benzoesäure.
(Fp.: 40-45°C)

### 2-Chlor-3-(2-methyl-2H-1,3,4-dioxazol-5-yl)-4-methylsulfonyl-benzoesäuremethylester (nicht efindungsgemäß)

Zu 8,0 g (27,4 mmol) 2-Chlor-3-(hydroxyiminomethyl)-4-methylsulfonyl-benzoesäuremethylester in 150 ml Methylenchlorid wurden 16,0 g (27,4 mmol) einer 12,5 %igen Natriumhypochloritlösung getropft und eine Spatelspitze Natriumacetat zugegeben. Nach 1 Stunde wurden innerhalb von 36 Stunden in Portionen 34,4 g (0,74 mol) Acetaldehyd zugegeben und langsam bis auf 55°C erhitzt. Anschließend wurde 48 Stunden bei Raumtemperatur gerührt, mit Wasser gewaschen, getrocknet und eingeengt. Der Rückstand wurde dann in Methylenchlorid aufgenommen, 10,0 g (0,23 mol) Acetaldehyd und eine Spatelspitze Natriumacetat zugegeben und 8 Stunden unter Rückfluß erhitzt. Nach 72 Stunden wurden nochmals 10,0 g (0,23 mol) Acetaldehyd zugegeben und bei Raumtemperatur gerührt. Anschließend wurde mit Wasser gewaschen, getrocknet und eingeengt. Der Rückstand wurde an Kieselgel (Eluent: Isopropanol Cyclohexan = 1:9) eluiert. Man erhielt 5,0 g (55 % d.Th.) 2-Chlor-3-(2-methyl-2H-1,3,4-dioxazol-5-yl)-4-methylsulfonyl-benzoesäuremethylester.

In der nachfolgenden Tabelle 4 sind weitere Beazoesdurederivate der Formel III aufgeführt, die in analoger Weise hergestellt wurden oder herstellbar sind.

**Tabelle 4:**

| | | | | | | | | | |
|---|---|---|---|---|---|---|---|---|---|
| 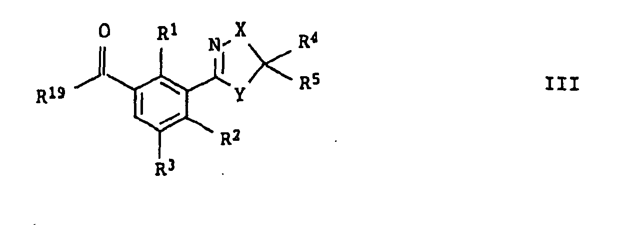 | | | | | | | | | |

| Nr. | R¹ | R² | R³ | X | R⁴ | R⁵ | Y | R¹⁹ | physikalische Daten Fp. [°C]; ¹H-NMR [δ in ppm] |
|---|---|---|---|---|---|---|---|---|---|
| 4.85 | SO₂CH₃ | SO₂CH₃ | H | O | H | H | CH₂ | OH | 3,25 (s); 3,35 (s); 3,44 (t); 8,05 (d); 8,45 (d). |

Die 3-Heterocycly-substituierten Benzoylderivate der Formel I/Ib23 und deren landwirtschaftlich brauchbaren Salze eignen sich - sowohl als Isomerengemische als auch in Form der reinen Isomeren - als Herbizide. Die Verbindungen der Formel I/Ib23 enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I/Ib23 bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis. Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa . Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I/Ib23 auch in Kulturen, die durch Züchtung einschließlich gentechnischer Methoden gegen die Wirkung von Herbiziden tolerant sind, verwandt werden.

Die Verbindungen der Formel I/Ib23 bzw. die sie enthaltenden herbiziden Mittel können beispielsweise in Form von direkt versprühbaren wäßrigen Lösungen, Pulvern; Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I/Ib23 oder eines landwirtschaftlich brauchbaren Salzes von I/Ib23 und für die Formulierung von Pflanzenschutzmitteln übliche Hilfsmittel.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I/Ib23 in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 3.2 (nicht erfindungsgemäß) werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wässrige Dispersion, die 0,02 Gew.-% des Wirkstoffs enthält.
II. 20 Gewichtsteile der Verbindung Nr. 3.9 (nicht erfindungsgemäß) werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 3.10 (nicht erfindungsgemäß) werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 3.16 (nicht erfindungsgemäß) werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 3.21(nicht erfindungsgemäß) werden mit 97 Gewichtsteilen feinteiligem Kaolin vermischt. Man erhält auf diese Weise ein Stäubemittel, das 3 Gew.-% des wirkstoffs enthält.
VI. 20 Gewichtsteile des Wirkstoffs Nr. 3.22 (nicht erfndungsgemäß) werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 3.34(nicht erfindungsgemäß) wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 3.35 (nicht erfindungsgemäß) wird in einer Mischung gelöst, die aus 80 Gewichtsteilen Cyclohexanon und 20 Gewichtsteilen Wettol^{®} EM 31 (= nichtionischer Emulgator auf der Basis von ethoxyliertem Rizinusöl) besteht. Man erhält ein stabiles Emulsionskonzentrat.

Die Applikation der Verbindungen der Formel I/Ib23bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschte Pflanzen oder die unbedeckte Bodenfläche gelangen (post-directed, lay-by).

Die Aufwandmengen an Verbindung der Formel I/Ib23 betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 3-Heterocyclyl-substituierten Benzoylderivate der Formel I/Ib23 mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I/Ib23 allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährung- und Spurenelementmängeln eingesetzt werden. Es können auch nichtphytotoxische Öle und Ölkonzentrate zugesetzt werden.

### Anwendungsbeispiele

Die herbizide Wirkung der 3-Heterocyclyl-substituierten Benzoylderivate der Formel I/Ib23 ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 31,2 bzw. 15,6 g/ha a.S. (aktive Substanz).

Die Pflanzen wurden artenspezifisch bei Temperaturen von 10 bis 25°C bzw. 20 bis 36°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Setaria faberii | Borstenhirse | giant foxtail |
| Sinapsis alba | weißer Senf | white mustard |
| Solanum nigrum | Schwarzer Nachtschatten | black nightshade |
| Triticum aestivum | Winterweizen | winter wheat |
| Zea mays | Mais | Indian corn |

## Patentansprüche

1. 3-Heterocyclyl-substituierte Benzoylderivate der Formel I 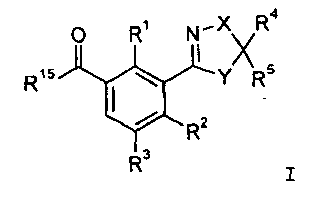 in der die Variablen folgende Bedeutungen haben:
R¹ Nitro, C₁-C₆-Alkyl, C₁-C₆-Alkoxy, C₁-C₆-Halogenalkyl, C₁-C₆-Alkylsulfonyl, C₁-C₆-Halogenalkylsulfonyl;
R² Nitro, Halogen, C₁-C₆-Alkyl, C₁-C₆-Halogenalkyl, C₁-C₆-Alkylthio, C₁-C₆-Alkylsulfinyl, C₁-C₆-Alkylsulfonyl, C₁-C₆-Halogenalkylsulfonyl;
R³ Wasserstoff;
R⁴, R⁵ Wasserstoff;
X Sauerstoff
Y CR¹³R¹⁴
R¹³, R¹⁴ Wasserstoff, C₁-C₄-Alkyl, C₁-C₄-Halogenalkyl, C₁-C₄-Alkoxycarbonyl, C₁-C₄-Halogenalkoxycarbonyl, CONR⁷R⁸, mit R⁷ in der Bedeutung von Wasserstoff oder C₁-C₄-Alkyl und R⁸ in der Bedeutung von C₁-C₄-Alkyl;
R¹⁵ ein in 4-Stellung verknüpftes Pyrazol der Formel II 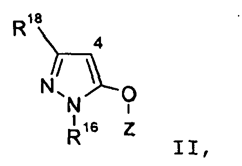 wobei
R¹⁶ C₁-C₆-Alkyl
Z Wasserstoff;
R¹⁸ Wasserstoff;
bedeuten;
sowie deren landwirtschaftlich brauchbaren Salze.

2. 3-Heterocyclyl-substituierte Benzoylderivate der Formel I nach Anspruch 1, in der die Variablen folgende Bedeutungen haben:
R¹ Methyl;
R² Methylsulfonyl;
R¹⁶ Methyl;
sowie deren landwirtschaftlich brauchbaren Salze.

3. 3-Heterocyclyl-substituierte Benzoylderivate der Formel Ib23, 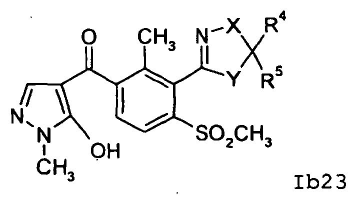 in der die Variablen folgende Bedeutungen haben:
| Nr. | X | R⁴ | R⁵ | Y |
|---|---|---|---|---|
| Ib23.1 | CH₂ | H | CH₃ | O |
| Ib23.2 | CH₂ | H | H | O |
| Ib23.3 | C(CH₃)₂ | H | H | O |
| Ib23.4 | CH₂ | H | C₂H₅ | O |
| Ib23.5 | CH₂ | CH₃ | CH₃ | O |
| Ib23.6 | CH(CH₃) | H | CH₃ | O |
| Ib23.7 | CH(C₂H₅) | H | CH₃ | O |
| Ib23.8 | CH [CH (CH₃)₂] | H | H | O |
| Ib23.9 | CH₂ | H | CH (CH₃)₂ | O |
| Ib23.10 | CH(C₂H₅) | H | C₂H₅ | O |
| Ib23.11 | -CH-(CH₂)₄- | | H | O |
| Ib23.12 | C=O | CH₃ | CH₃ | O |
| Ib23.13 | C=O | H | C₂H₅ | O |
| Ib23.14 | C=O | C₂H₅ | C₂H₅ | O |
| Ib23.15 | C=O | H | H | O |
| Ib23.16 | C=O | H | CH₃ | O |
| Ib23.17 | CH₂ | H | CH₃ | S |
| Ib23.18 | CH₂ | H | H | S |
| Ib23.19 | C (CH₃)₂ | H | H | S |
| Ib23.20 | CH₂ | H | C₂H₅ | S |
| Ib23.21 | CH₂ | CH₃ | CH₃ | S |
| Ib23.22 | CH (CH₃) | H | CH₃ | S |
| Ib23.23 | CH (C₂H₅) | H | CH₃ | S |
| Ib23. 24 | CH (C₂H₅) | H | C₂H₅ | S |
| Ib23.25 | -CH-(CH₂)₄- | | H | S |
| Ib23.26 | CH[CH(CH₃)₂] | H | H | S |
| Ib23.27 | CH₂ | H | CH (CH₃)₂ | S |
| Ib23.28 | CH₂ | H | CH₃ | NH |
| Ib23.29 | CH₂ | H | H | NH |
| Ib23. 30 | C(CH₃)₂ | H | H | NH |
| Ib23.31 | CH₂ | H | C₂H₅ | NH |
| Ib23.32 | CH₂ | CH₃ | CH₃ | NH |
| Ib23. 33 | CH(CH₃) | H | CH₃ | NH |
| Ib23.34 | CH(C₂H₅) | H | CH₃ | NH |
| Ib23.35 | CH (C₂H₅) | H | C₂H₅ | NH |
| Ib23. 36 | -CH- (CH₂)₄- | | H | NH |
| Ib23.37 | CH[CH(CH₃)₂] | H | H | NH |
| Ib23.38 | CH₂ | H | CH(CH₃)₂ | NH |
| Ib23.39 | CH₂ | H | CH₃ | NCH₃ |
| Ib23.40 | CH₂ | H | H | NCH₃ |
| Ib23.41 | C (CH₃)₂ | H | H | NCH₃ |
| Ib23.42 | CH₂ | H | C₂H₅ | NCH₃ |
| Ib23.43 | CH₂ | CH₃ | CH₃ | NCH₃ |
| Ib23.44 | CH(CH₃) H | | CH₃ | NCH₃ |
| Ib23.45 | CH (C₂H₅) | H | CH₃ | NCH₃ |
| Ib23.46 | CH[CH(CH₃)₂] | H | H | NCH₃ |
| Ib23.47 | CH₂ | H | CH (CH₃)₂ | NCH₃ |
| Ib23. 48 | CH (C₂H₅) | H | C₂H₅ | NCH₃ |
| Ib23.49 | -CH-(CH₂)₄- | | H | NCH₃ |
| Ib23.50 | CH₂ | H | CH₃ | NC₂H₅ |
| Ib23.51 | CH₂ | H | H | NC₂H₅ |
| Ib23.52 | C(CH₃)₂ | H | H | NC₂H₅ |
| Ib23.53 | CH₂ | H | C₂H₅ | NC₂H₅ |
| Ib23.54 | CH₂ | CH₃ | CH₃ | NC₂H₅ |
| Ib23. 55 | CH(CH₃) | H | CH₃ | NC₂H₅ |
| Ib23.56 | CH (C₂H₅) | H | CH₃ | NC₂H₅ |
| Ib23.57 | CH [CH (CH₃)₂] | H | H | NC₂H₅ |
| Ib23.58 | CH₂ | H | CH(CH₃)₂ | NC₂H₅ |
| Ib23. 59 | CH (C₂H₅) | H | C₂H₅ | NC₂H₅ |
| Ib23.60 | -CH- (CH₂)₄- | | H | NC₂H₅ |
| Ib23.61 | CH₂ | =O | | S |
| Ib23.62 | CH (CH₃) | =O | | S |
| Ib23.63 | CH (C₂H₅) | =O | | S |
| Ib23.64 | CH [CH (CH₃) ₂] | =O | | S |
| Ib23.65 | C(CH₃) ₂ | =O | | S |
| Ib23.66 | CCH₃ (C₂H₅) | =O | | S |
| Ib23.67 | CCH₃ [CH (CH₃)₂] | =O | | S |
| Ib23.68 | CH₂ | =O | | NH |
| Ib23.69 | CH(CH₃) | =O | | NH |
| Ib23.70 | CH(C₂H₅) | =O | | NH |
| Ib23.71 | CH [CH (CH₃)₂] | =O | | NH |
| Ib23.72 | C(CH₃)₂ | =O | | NH |
| Ib23.73 | CCH₃(C₂H₅) | =O | | NH |
| Ib23.74 | CCH₃[CH(CH₃)₂] | =O | | NH |
| Ib23.75 | CH₂ | =O | | NCH₃ |
| Ib23.76 | CH(CH₃) | =O | | NCH₃ |
| Ib23.77 | CH (C₂H₅) | =O | | NCH₃ |
| Ib23.78 | CH [CH ( CH₃)₂] | =O | | NCH₃ |
| Ib23.79 | C(CH₃)₂ | =O | | NCH₃ |
| Ib23.80 | CCH₃(C₂H₅) | =O | | NCH₃ |
| Ib23.81 | CCH₃[CH(CH₃)₂] | =O | | NCH₃ |
| Ib23.82 | O | COOCH₃ | H | CH₂ |
| Ib23.83 | O | COOC₂H₅ | H | CH₂ |
| Ib23.84 | O | CONHCH₃ | H | CH₂ |
| Ib23.85 | O | CON (CH₃)₂ | H | CH₂ |
| Ib23.86 | O | CONHC₂H₅ | H | CH₂ |
| Ib23.87 | O | CON (C₂H₅)₂ | H | CH₂ |
| Ib23.88 | O | CH₃ | H | CH₂ |
| Ib23.89 | O | C₂H₅ | H | CH₂ |
| Ib23.90 | O | CH (CH₃)₂ | H | CH₂ |
| Ib23.91 | O | COC₂H₅ | H | CH₂ |
| Ib23.92 | O | CH₂CN | H | CH₂ |
| Ib23.93 | O | CH₂N (CH₃)₂ | H | CH₂ |
| Ib23.94 | O | CH₂ON=C (CH₃)₂ | H | CH₂ |
| Ib23.95 | O | CH (OC₂H₅)₂ | H | CH₂ |
| Ib23.96 | O | CH(OCH₃)₂ | H | CH₂ |
| Ib23.97 | O | CH₃ | CH₃ | CH₂ |
| Ib23.98 | O | CH₃ | C₂H₅ | CH₂ |
| Ib23.99 | O | C₂H₅ | C₂H₅ | CH₂ |
| Ib23.100 | O | -(CH₂)₄- | | CH₂ |
| Ib23.101 | O | -(CH₂)₂-O-(CH₂)₂- | | CH₂ |
| Ib23.102 | O | H | - (CH₂)₃-CH- | |
| Ib23.103 | O | H | -(CH₂)₄-CH- | |
| Ib23.104 | O | CH₃ | H | CHCH₃ |
| Ib23.105 | O | H | H | CH₂ |
| Ib23.106 | S | =O | | O |
| Ib23.107 | CH₂ | =S | | S |
| Ib23.108 | CH(CH₃) | =S | | S |
| Ib23.109 | CH (C₂H₅) | =S | | S |
| Ib23.110 | C(CH₃)₂ | =S | | S |
| Ib23.111 | O | =O | | NH |
| Ib23.112 | O | =O | | NCH₃ |
| Ib23.113 | O | CH₃ | H | NH |
| Ib23.114 | O | C₂H₅ | H | NH |
| Ib23.115 | O | CH₃ | CH₃ | NH |
| Ib23.116 | O | C₂H₅ | C₂H₅ | NH |
| Ib23.117 | O | CH₃ | H | NCH₃ |
| Ib23.118 | O | C₂H₅ | H | NCH₃ |
| Ib23.119 | O | CH₃ | CH₃ | NCH₃ |
| Ib23.120 | O | C₂H₅ | C₂H₅ | NCH₃ |
| Ib23.121 | NH | =O | | NH |
| Ib23.122 | NH | =O | | NCH₃ |
| Ib23.123 | NCH₃ | =O | | NH |
| Ib23.124 | NCH₃ | =O | | NCH₃ |
| Ib23.125 | NC₂H₅ | =O | | NH |
| Ib23.126 | NC₂H₅ | =O | | NC₂H₅ |
sowie deren landwirtschaftlich brauchbaren Salze.

4. 4-[2-methyl-3-(4,5-dihydroisoxazol-3-yl)-4-methylsulfonylbenzoyl]-1-methyl-5-hydroxy-1H-pyrazol.

5. Landwirtschaftlich brauchbare Salze von 4-[2-methyl-3-(4,5-dihydroisoxazol-3-yl)-4-methylsulfonylbenzoyl]-1-methy1-5-hydroxy-1H-pyrazol.

6. Verfahren zur Herstellung von 3-Heterocyclyl-substituierten Benzoylderivaten der Formel I oder Ib23 gemäß Anspruch 1 bis 5, **dadurch gekennzeichnet, dass** man das Pyrazol der Formel II, wobei die Variablen R¹⁶ und R¹⁸ die unter den Ansprüchen 1 bis 5 genannten Bedeutungen haben, 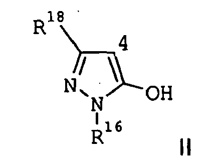 mit einer aktivierten Carbonsäure IIIα oder mit einer Carbonsäure IIIβ, 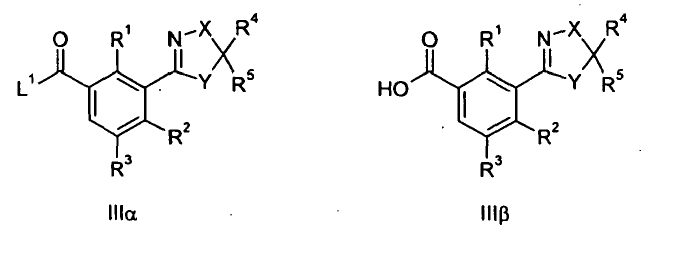 wobei die Variablen R¹ bis R⁵, X und Y die unter den Ansprüchen 1 bis 5 genannten Bedeutungen haben und L¹ für eine nucleophil verdrängbare Abgangsgruppe steht, acyliert und das Acylierungsprodukt, gegebenenfalls in Gegenwart eines Katalysators, zu den Verbindungen I oder Ib23 umlagert.

7. 3-Heterocycly1-substituierte Benzoesäurederivate der Formel III, 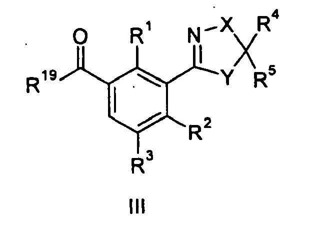 wobei R¹⁹ für Hydroxy oder einen abhydrolysierbaren Rest steht und die Variablen R¹ bis R⁵, X und Y die unter den Ansprüchen 1 bis 5 genannte Bedeutung haben.

8. 3-Heterocyclyl-substituierte Benzoesäurederivate der Formel III nach Anspruch 7, wobei
R¹⁹ Halogen, Hydroxy oder C₁-C₆-Alkoxy bedeutet.

9. Mittel, enthaltend eine herbizid wirksame Menge mindestens eines 3-Heterocyclyl-substituierten Benzoylderivats der Formel 1 oder Ib23 oder eines landwirtschaftlich brauchbaren Salzes von I oder Ib23 gemäß den Ansprüchen 1 bis 5, und für die Formulierung von Pflanzenschutzmitteln übliche Hilfsmittel.

10. Verfahren zur Herstellung von Mitteln gemäß Anspruch 9, **dadurch gekennzeichnet, dass** man eine herbizid wirksame Menge mindestens eines 3-Heterocyclyl-substituierten Benzoylderivats der Formel I oder Ib23 oder eines landwirtschaftlich brauchbaren Salzes von I oder Ib23 gemäß den Ansprüchen 1 bis 5 und für die Formulierung von Pflanzenschutzmitteln übliche Hilfsmittel mischt.

11. Verfahren zur Bekämpfung von unerwünschtem Pflanzenwuchs, **dadurch gekennzeichnet, dass** man eine herbizid wirksame Menge mindestens eines 3-Heterocyclyl-substituierten Benzoylderivats der Formel I oder Ib23 oder eines landwirtschaftlich brauchbaren Salzes von I oder Ib23 gemäß den Ansprüchen 1 bis 5, auf Pflanzen, deren Lebensraum und/oder auf Samen einwirken läßt.

12. Verwendung der 3-Heterocyclyl-substituierten Benzoylderviate der Formel I oder Ib23 und deren landwirtschaftlich brauchbaren Salzen gemäß den Ansprüchen 1 bis 5 als Herbizide.

## Claims

1. 3-(4,5-Dihydroisoxazol-3-yl)-substituted benzoyl derivatives of the formula I 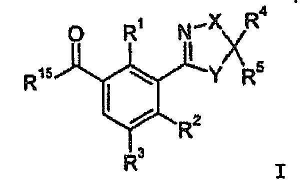 in which the variables have the following meanings:
R¹ is nitro, C₁-C₆-alkyl, C₁-C₆-alkoxy, C₁-C₆-haloalkyl, C₁-C₆-alkylsulphonyl, C₁-C₆-haloalkylsulphonyl;
R² is nitro, halogen, C₁-C₆-alkyl, C₁-C₆-haloalkyl, C₁-C₆-alkylthio, C₁-C₆-alkylsulphynyl, C₁-C₆-alkylsulphonyl , C₁-C₆-haloalkyl-sulphonyl;
R³ is hydrogen;
R⁴, R⁵ are hydrogen;
X is oxygen;
Y is CR¹³R¹⁴;
R¹³,R¹⁴ are hydrogen, C₁-C₄-alkyl, C₁-C₄-haloalkyl, C₁-C₄-alkoxycarbonyl, C₁-C₄-haloalkoxy-carbonyl, CONR⁷ R⁸, where R⁷ has the meaning of hydrogen or C₁-C₄-alkyl and R⁸ has the meaning of C₁-C₄-alkyl;
R¹⁵ is a pyrazole of the formula II which is linked in the 4-position 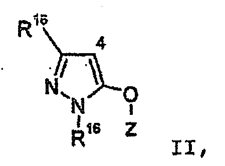 where
R¹⁶ is C₁-C₆-alkyl;
Z is hydrogen;
R¹⁸ is hydrogen;
and their agriculturally useful salts.

2. 3-(4,5-Dihydroisoxazol-3-yl)-substituted benzoyl derivatives of the formula I according to Claim 1 in which the variables have the following meanings:
R¹ is methyl;
R² is methylsulphonyl;
R¹⁶ is methyl;
and their agriculturally useful salts.

3. 3-Heterocyclyl-substituted benzoyl derivatives of the formula Ib23, 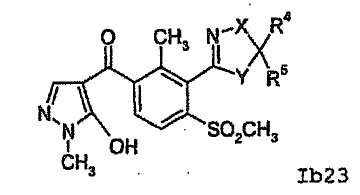 in which the variables have the following meanings:
| No. | X | R⁴ | R⁵ | Y |
|---|---|---|---|---|
| Ib23.1 | CH₂ | H | CH₃ | O |
| Ib23.2 | CH₂ | H | H | O |
| Ib23.3 | C(CH₃)₂ | H | H | O |
| Ib23.4 | CH₂ | H | C₂H₅ | O |
| Ib23.5 | CH₂ | CH₃ | CH₃ | O |
| Ib23.6 | CH(CH₃) | H | CH₃ | O |
| Ib23.7 | CH(C₂H₅) | H | CH₃ | O |
| Ib23.8 | CH[CH(CH₃)₂] | H | H | O |
| Ib23.9 | CH₂ | H | CH ( CH₃)₂ | O |
| Ib23.10 | CH(C₂H₅) | H | C₂H₅ | O |
| Ib23.11 | -CH-(CH₂)₄- | | H | O |
| Ib23.12 | C=O | CH₃ | CH₃ | O |
| Ib23.13 | C=O | H | C₂H₅ | O |
| Ib23.14 | C=O | C₂H₅ | C₂H₅ | O |
| Ib23.15 | C=O | H | H | O |
| Ib23.16 | C=O | H | CH₃ | O |
| Ib23.17 | CH₂ | H | CH₃ | S |
| Ib23.18 | CH₂ | H | H | S |
| Ib23.19 | C(CH₃)₂ | H | H | S |
| Ib23.20 | CH₂ | H | C₂H₅ | S |
| Ib23.21 | CH₂ | CH₃ | CH₃ | S |
| Ib23.22 | CH (CH₃) | H | CH₃ | S |
| Ib23.23 | CH (C₂H₅) | H | CH₃ | S |
| Ib23.24 | CH(C₂H₅) | H | C₂H₅ | S |
| Ib23.25 | -CH- (CH₂)₄- | | H | S |
| Ib23.26 | CH [CH(CH₃)₂] | H | H | S |
| Ib23.27 | CH₂ | H | CH (CH₃)₂ | S |
| Ib23.28 | CH₂ | H | CH₃ | NH |
| Ib23.29 | CH₂ | H | H | NH |
| Ib23.30 | C(CH₃)₂ | H | H | NH |
| Ib23.31 | CH₂ | H | C₂H₅ | NH |
| Ib23.32 | CH₂ | CH₃ | CH₃ | NH |
| Ib23.33 | CH(CH₃) | H | CH₃ | NH |
| Ib23.34 | CH(C₂H₅) | H | CH₃ | NH |
| Ib23.35 | CH(C₂H₅) | H | C₂H₅ | NH |
| Ib23.36 | -CH-(CH₂)₄- | | H | NH |
| Ib23.37 | CH [CH(CH₃)₂] | H | H | NH |
| Ib23.38 | CH₂ | H | CH(CH₃)₂ | NH |
| Ib23.39 | CH₂ | H | CH₃ | NCH₃ |
| Ib23.40 | CH₂ | H | H | NCH₃ |
| Ib23.41 | C(CH₃)₂ | H | H | NCH₃ |
| Ib23.42 | CH₂ | H | C₂H₅ | NCH₃ |
| Ib23.43 | CH₂ | CH₃ | CH₃ | NCH₃ |
| Ib23.44 | CH (CH₃) | H | CH₃ | NCH₃ |
| Ib23.45 | CH(C₂H₅ ) | H | CH₃ | NCH₃ |
| Ib23.46 | CH [CH (CH₃)₂] | H | H | NCH₃ |
| Ib23.47 | CH₂ | H | CH(CH₃)₂ | NCH₃ |
| Ib23.48 | CH(C₂H₅) | H | C₂H₅ | NCH₃ |
| Ib23.49 | -CH-(CH₂)₄- | | H | NCH₃ |
| Ib23.50 | CH₂ | H | CH₃ | NC₂R₅ |
| Ib23.51 | CH₂ | H | H | NC₂H₅ |
| Ib23.52 | C (CH₃)₂ | H | H | NC₂H₅ |
| Ib23.53 | CH₂ | H | C₂H₅ | NC₂H₅ |
| Ib23.54 | CH₂ | CH₃ | CH₃ | NC₂H₅ |
| Ib23.55 | CH (CH₃) | H | CH₃ | NC₂H₅ |
| Ib23.56 | CH (C₂H₅) | H | CH₃ | NC₂H₅ |
| Ib23.57 | CH[CH(CH₃)₂] | H | H | NC₂H₅ |
| Ib23.58 | CH₂ | H | CH (CH₃)₂ | NC₂H₅ |
| Ib23. 59 | CH (C₂H₅) | H | C₂H₅ | NC₂H₅ |
| Ib23.60 | -CH-(CH₂)₄- | | H | NC₂H₅ |
| Ib23.61 | CH₂ | =O | | S |
| Ib23.62 | CH (CH₃) | =O | | S |
| Ib23.63 | CH (C₂H₅) | =O | | S |
| Ib23.64 | CH[CH(CH₃)₂] | =O | | S |
| Ib23.65 | C (CH₃)₂ | =O | | S |
| Ib23.66 | CCH₃ (C₂H₅) | =O | | S |
| Ib23.67 | CCH₃[CH(CH₃)₂] | =O | | S |
| Ib23.68 | CH₂ | =O | | NH |
| Ib23.69 | CH (CH₃) | =O | | NH |
| Ib23.70 | CH(C₂H₅) | =O | | NH |
| Ib23.71 | CH[CH(CH₃)₂] | =O | | NH |
| Ib23.72 | C(CH₃)₂ | =O | | NH |
| Ib23.73 | CCH₃(C₂H₅) | =O | | NH |
| Ib23.74 | CCH₃[CH(CH₃)₂] | =O | | NH |
| Ib23.75 | CH₂ | =O | | NCH₃ |
| Ib23.76 | CH(CH₃) | =O | | NCH₃ |
| Ib23.77 | CH(C₂H₅) | =O | | NCH₃ |
| Ib23.78 | CH [CH(CH₃)₂] | =O | | NCH₃ |
| Ib23.79 | C(CH₃)₂ | =O | | NCH₃ |
| Ib23.80 | CCH₃(C₂H₅) | =O | | NCH₃ |
| Ib23.81 | CCH₃[CH(CH₃)₂] | =O | | NCH₃ |
| Ib23.82 | O | COOCH₃ | H | CH₂ |
| Ib23.83 | O | COOC₂H₅ | H | CH₂ |
| Ib23.84 | O | CONHCH₃ | H | CH₂ |
| Ib23.85 | O | CON (CH₃)₂ | H | CH₂ |
| Ib23.86 | O | CONHC₂H₅ | H | CH₂ |
| Ib23.87 | O | CON (C₂H₅)₂ | H | CH₂ |
| Ib23.88 | O | CH₃ | H | CH₂ |
| Ib23.89 | O | C₂H₅ | H | CH₂ |
| Ib23.90 | O | CH(CH₃)₂ | H | CH₂ |
| Ib23.91 | O | COC₂H₅ | H | CH₂ |
| Ib23.92 | O | CH₂CN | H | CH₂ |
| Ib23.93 | O | CH₂N(CH₃)₂ | H | CH₂ |
| Ib23.94 | O | CH₂ON=C (CH₃)₂ | H | CH₂ |
| Ib23.95 | O | CH(OC₂H₅)₂ | H | CH₂ |
| Ib23.96 | O | CH (OCH₃)₂ | H | CH₂ |
| Ib23.97 | O | CH₃ | CH₃ | CH₂ |
| Ib23.98 | O | CH₃ | C₂H₅ | CH₂ |
| Ib23.99 | O | C₂H₅ | C₂H₅ | CH₂ |
| Ib23.100 | O | - (CH₂)₄- | | CH₂ |
| Ib23.101 | O | - (CH₂)₂-O- (CH₂)₂- | | CH₂ |
| Ib23.102 | O | H | - (CH₂)₃-CH- | |
| Ib23.103 | O | H | - (CH₂)₄-CH- | |
| Ib23.104 | O | CH₃ | H | CHCH₃ |
| Ib23.105 | O | H | H | CH₂ |
| Ib23.106 | S | =O | | O |
| Ib23.107 | CH₂ | =S | | S |
| Ib23.108 | CH(CH₃) | =S | | S |
| Ib23.109 | CH(C₂H₅) | =S | | S |
| Ib23.110 | C(CH₃)₂ | =S | | S |
| Ib23.111 | O | =O | | NH |
| Ib23.112 | O | =O | | NCH₃ |
| Ib23.113 | O | CH₃ | H | NH |
| Ib23.114 | O | C₂H₅ | H | NH |
| Ib23.115 | O | CH₃ | CH₃ | NH |
| Ib23.116 | O | C₂H₅ | C₂H₅ | NH |
| Ib23.117 | O | CH₃ | H | NCH₃ |
| Ib23.118 | O | C₂H₅ | H | NCH₃ |
| Ib23.119 | O | CH₃ | CH₃ | NCH₃ |
| Ib23.120 | O | C₂H₅ | C₂H₅ | NCH₃ |
| Ib23.121 | NH | =O | | NH |
| Ib23.122 | NH | =O | | NCH₃ |
| Ib23.123 | NCH₃ | =O | | NH |
| Ib23.124 | NCH₃ | =O | | NCH₃ |
| Ib23.125 | NC₂H₅ | =O | | NH |
| Ib23.126 | NC₂H₅ | =O | | NC₂H₅ |
and their agriculturally useful salts.

4. 4-[2-Methyl-3-(4,5-dihydroisoxazol-3-yl)-4-methylsulphonylbenzoyl]-1-methyl-5-hydroxy-1H-pyrazole.

5. Agriculturally useful salts of 4-[2-methyl-3-(4,5-dihydroisoxazol-3-yl)-4-methylsulphonylbenzoyl]-1-methyl-5-hydroxy-1H-pyrazole.

6. Process for the preparation of 3-heterocyclyl-substituted benzoyl derivatives of the formula I or Ib23 according to Claim 1 to 5, **characterized in that** the pyrazole of the formula II, where the variables R¹⁶ and R¹⁸ have the meanings mentioned in Claims 1 to 5, 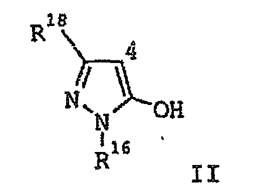 are acylated with an activated carboxylic acid IIIα or with a carboxylic acid IIIβ, 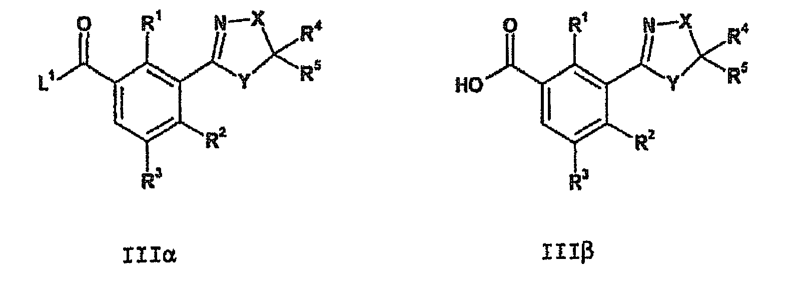 where the variables R¹ to R⁵, X and Y have the meanings mentioned in Claims 1 to 5 and L¹ represents a nucleophilically displaceable leaving group, and the acylation product is subjected to a rearrangement reaction, if appropriate in the presence of a catalyst, to give the compounds I or Ib23.

7. 3-Heterocyclyl-substituted benzoic acid derivatives of the formula III, 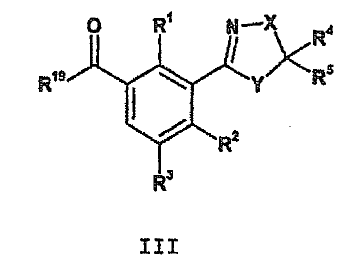 where R¹⁹ is hydroxyl or a radical which can be removed hydrolytically, and the variables R¹ to R⁵, X and Y have the meanings mentioned in Claims 1 to 5.

8. 3-Heterocyclyl-substituted benzoic acid derivatives of the formula III according to Claim 7, where
R¹⁹ is halogen, hydroxyl or C₁-C₆-alkoxy.

9. Composition, comprising a herbicidally active amount of at least one 3-heterocyclyl-substituted benzoyl derivative of the formula I or Ib23 or of an agriculturally useful salt of I or Ib23 according to Claims 1 to 5, and adjuvants which are conventionally used for the formulation of plant protection products.

10. Process for the preparation of compositions according to Claim 9, **characterized in that** a herbicidally active amount of at least one 3-heterocyclyl-substituted benzoyl derivative of the formula I or Ib23 or of an agriculturally useful salt of I or Ib23 according to Claims 1 to 5 and adjuvants which are conventionally used for the formulation of plant protection products are mixed with each other.

11. Method of controlling undesirable vegetation, **characterized in that** a herbicidally active amount of at least one 3-heterocyclyl-substituted benzoyl derivative of the formula I or Ib23 or of an agriculturally useful salt of I or Ib23 according to Claims 1 to 5 is allowed to act on plants, their environment and/or on seeds.

12. Use of the 3-heterocyclyl-substituted benzoyl derivatives of the formula I or Ib23 and of their agriculturally useful salts according to Claims 1 to 5 as herbicides.

## Revendications

1. Dérivés de benzoyle à substitution 3-(4,5-dihydroisoxazol-3-yle) de formule I : 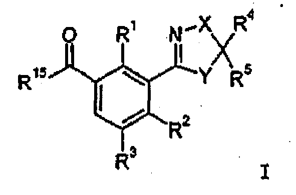 dans laquelle les variables ont les significations suivantes :
R¹ représente un nitro, un groupement alkyle en C₁-C₆, alcoxy en C₁-C₆, halogénoalkyle en C₁-C₆, alkyl-sulfonyle en C₁-C₆, halogénoalkylsulfonyle en C₁-C₆ ;
R² représente un nitro, un halogène, un groupement alkyle en C₁-C₆, halogénoalkyle en C₁-C₆, alkylthio en C₁-C₆, alkylsulfinyle en C₁-C₆, alkylsulfonyle en C₁-C₆, halogénoalkylsulfonyle en C₁-C₆ ;
R³ représente l'hydrogène ;
R⁴, R⁵ représente l'hydrogène ;
X représente l'oxygène ;
Y représente CR¹³R¹⁴
R¹³, R¹⁴ représentent l'hydrogène, un groupement alkyle en C₁-C₄, halogénoalkyle en C₁-C₄, alcoxycarbonyle en C₁-C₄, halogénoalcoxycarbonyle en C₁-C₄, CONR⁷R⁸, où R⁷ représente l'hydrogène ou un alkyle en C₁-C₄ et R⁸ représente un alkyle en C₁-C₄ ;
R¹⁵ représente un pyrazole relié via la position 4, de formule II : 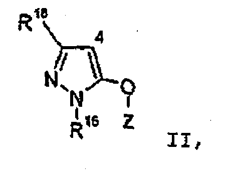 dans laquelle :
R¹⁶ représente un alkyle en C₁-C₆ ;
Z représente l'hydrogène ;
R¹⁸ représente l'hydrogène ;
ainsi que leurs sels utilisables en agriculture.

2. Dérivés de benzoyle à substitution 3-(4,5-dihydroisoxazol-3-yle) de formule I selon la revendication 1, dans laquelle les variables ont les significations suivantes :
R¹ représente un méthyle ;
R² représente un méthylsulfonyle ;
R¹⁶ représente un méthyle ;
ainsi que leurs sels utilisables en agriculture.

3. Dérivés de benzoyle à substitution 3-hétérocyclyle de formule Ib23 suivante: 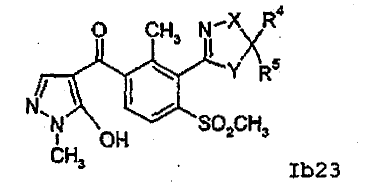 dans laquelle les variables ont les significations suivantes :
| N° | X | R⁴ | R⁵ | Y |
|---|---|---|---|---|
| Ib23.1 | CH₂ | H | CH₃ | O |
| Ib23.2 | CH₂ | H | H | O |
| Ib23.3 | C(CH₃)₂ | H | H | O |
| Ib23.4 | CH₂ | H | C₂H₅ | O |
| Ib23.5 | CH₂ | CH₃ | CH₃ | O |
| Ib23.6 | CH(CH₃) | H | CH₃ | O |
| Ib23.7 | CH(C₂H₅) | H | CH₃ | O |
| Ib23.8 | CH[CH(CH₃)₂] | H | H | O |
| Ib23.9 | CH₂ | H | CH(CH₃)₂ | O |
| Ib23.10 | CH(C₂H₅ | H | C₂H₅ | O |
| Ib23.11 | -CH-(CH₂)₄- | | H | O |
| Ib23.12 | C=O | CH₃ | CH₃ | O |
| Ib23.13 | C=O | H | C₂H₅ | O |
| Ib23.14 | C=O | C₂H₅ | C₂H₅ | O |
| Ib23.15 | C=O | H | H | O |
| Ib.23.16 | C=O | H | CH₃ | O |
| Ib23.17 | CH₂ | H | CH₃ | S |
| Ib23.18 | CH₂ | H | H | S |
| IB23.19 | C(CH₃)₂ | H | H | S |
| Ib23.20 | CH₂ | H | C₂H₅ | S |
| Ib23.21 | CH₂ | CH₃ | CH₃ | S |
| Ib23.22 | CH(CH₃) | H | CH₃ | S |
| Ib23.23 | CH(C₂H₅) | H | CH₃ | S |
| Ib23.24 | CH (C₂H₅) | H | C₂H₅ | S |
| Ib23.25 | -CH-(CH₂)₄- | | H | S |
| Ib23.26 | CH[CH(CH₃)₂] | H | H | S |
| Ib23.27 | CH₂ | H | CH(CH₃)₂ | S |
| Ib23.28 | CH₂ | H | CH₃ | NH |
| Ib23.29 | CH₂ | H | H | NH |
| Ib23.30 | C(CH₃)₂ | H | H | NH |
| Ib23.31 | CH₂ | H | C₂H₅ | NH |
| Ib23.32 | CH₂ | CH₃ | CH₃ | NH |
| Ib23.33 | CH(CH₃) | H | CH₃ | NH |
| Ib23.34 | CH(C₂H₅) | H | CH₃ | NH |
| Ib23.35 | CH(C₂H₅) | H | C₂H₅ | NH |
| Ib23.36 | -CH-(CH₂)₄- | | H | NH |
| Ib23.37 | CH[CH(CH₃)₂] | H | H | NH |
| Ib23.38 | CH₂ | H | CH(CH₃)₂ | NH |
| Ib23.39 | CH₂ | H | CH₃ | NCH₃ |
| Ib23.40 | CH₂ | H | H | NCH₃ |
| Ib23.41 | C(CH₃)₂ | H | H | NCH₃ |
| Ib23.42 | CH₂ | H | C₂H₅ | NCH₃ |
| Ib23.43 | CH₂ | CH₃ | CH₃ | NCH₃ |
| Ib23.44 | CH(CH₃) | H | CH₃ | NCH₃ |
| Ib23.45 | CH(C₂H₅) | H | CH₃ | NCH₃ |
| Ib23.46 | CH[CH(CH₃)₂] | H | H | NCH₃ |
| Ib23.47 | CH₂ | H | CH(CH₃)₂ | NCH3 |
| Ib23.48 | CH(C₂H₅) | H | C₂H₅ | NCH₃ |
| Ib23.49 | -CH-(CH₂)₄- | | H | NCH₃ |
| Ib23.50 | CH₂ | H | CH₃ | NC₂H₅ |
| Ib23.51 | CH₂ | H | H | NC₂H₅ |
| Ib23.52 | C(CH₃)₂ | H | H | NC₂H₅ |
| Ib23.53 | CH₂ | H | C₂H₅ | NC₂H₅ |
| Ib23.54 | CH₂ | CH₃ | CH₃ | NC₂H₅ |
| Ib23.55 | CH (CH₃) | H | CH₃ | NC₂H₅ |
| Ib23.56 | CH (C₂H₅) | H | CH₃ | NC₂H₅ |
| Ib23.57 | CH[CH(CH₃)₂] | H | H | NC₂H₅ |
| Ib23.58 | CH₂ | H | CH(CH₃) | ₂ NC₂H₅ |
| Ib23.59 | CH (C₂H₅) | H | C₂H₅ | NC₂H₅ |
| Ib23.60 | -CH-(CH₂)₄- | | H | NC₂H₅ |
| Ib23.61 | CH₂ | =O | | S |
| Ib23.62 | CH(CH₃) | =O | | S |
| Ib23.63 | CH(C₂H₅) | =O | | S |
| Ib23.64 | CH[CH(CH₃)₂] | =O | | S |
| Ib23.65 | C(CH₃)₂ | =O | | S |
| Ib23.66 | CCH₃(C₂H₅) | =O | | S |
| Ib23.67 | CCH₃[CH(CH₃)₂] | =O | | S |
| Ib23.68 | CH₂ | =O | | NH |
| Ib23.69 | CH (CH₃) | =O | | NH |
| Ib23.70 | CH(C₂H₅) | =O | | NH |
| Ib23.71 | CH[CH(CH₃)₂] | =O | | NH |
| Ib23.72 | C(CH₃)₂ | =O | | NH |
| Ib23.73 | CCH₃(C₂H₅) | =O | | NH |
| Ib23.74 | CCH₃[CH(CH₃)₂] | =O | | NH |
| Ib23.75 | CH₂ | =O | | NCH₃ |
| Ib23.76 | CH(CH₃) | =O | | NCH₃ |
| Ib23.77 | CH(C₂H₅) | =O | | NCH₃ |
| Ib23.78 | CH[CH(CH₃)₂] | =O | | NCH₃ |
| Ib23.79 | C(CH₃)₂ | =O | | NCH3 |
| Ib23.80 | CCH₃(C₂H₅) | =O | | NCH₃ |
| Ib23.81 | CCH₃[CH(CH₃)₂] | =O | | NCH₃ |
| Ib23.82 | O | COOCH₃ | H | CH₂ |
| Ib23.83 | O | COOC₂H₅ | H | CH₂ |
| Ib23.84 | O | CONHCH₃ | H | CH₂ |
| Ib23.85 | O | CON(CH₃)₂ | H | CH₂ |
| Ib23.86 | O | CONHC₂H₅ | H | CH₂ |
| Ib23.87 | O | CON(C₂H₅)₂ | H | CH₂ |
| Ib23.88 | O | CH₃ | H | CH₂ |
| Ib23.89 | O | C₂H₅ | H | CH₂ |
| Ib23.90 | O | CH(CH₃)₂ | H | CH₂ |
| Ib23.91 | O | COC₂H₅ | H | CH₂ |
| Ib23.92 | 0 | CH₂CN | H | CH₂ |
| Ib23.93 | O | CH₂N(CH₃)₂ | H | CH₂ |
| Ib23.94 | O | CH₂ON-C(CH₃)₂ | H | CH₂ |
| Ib23.95 | O | CH(OC₂H₅)₂ | H | CH₂ |
| Ib23.96 | O | CH(OCH₃)₂ | H | CH₂ |
| Ib23.97 | O | CH₃ | CH₃ | CH₂ |
| Ib23.98 | O | CH₃ | C₂H₅ | CH₂ |
| Ib23.99 | O | C₂H₅ | C₂H₅ | CH₂ |
| Ib23.100 | O | -(CH₂)₄- | | CH₂ |
| Ib23.101 | O | -(CH₂)₂-O-(CH₂)₂- | | CH₂ |
| Ib23.102 | O | H | -(CH₂)₃-CH- | |
| Ib23.103 | O | H | -(CH₂)₄-CH- | |
| Ib23.104 | O | CH₃ | H | CHCH₃ |
| Ib23.105 | O | H | H | CH₂ |
| Ib23.106 | S | =O | | O |
| Ib23.107 | CH₂ | =S | | S |
| Ib23.108 | CH(CH₃) | =S | | S |
| Ib23.109 | CH(C₂H₅) | =S | | S |
| Ib23.110 | C(CH₃)₂ | =S | | S |
| Ib23.111 | O | =O | | NH |
| Ib23.112 | O | =O | | NCH₃ |
| Ib23.113 | O | CH₃ | H | NH |
| Ib23.114 | O | C₂H₅ | H | NH |
| Ib23.115 | O | CH₃ | CH₃ | NH |
| Ib23.116 | O | C₂H₅ | C₂H₅ | NH |
| Ib23.117 | O | CH₃ | H | NCH₃ |
| Ib23.118 | O | C₂H₅ | H | NCH₃ |
| Ib23.119 | O | CH₃ | CH₃ | NCH₃ |
| Ib23.120 | O | C₂H₅ C₂H₅ | C₂H₅ | NCH₃ |
| Ib23.121 | NH | =O | | NH |
| Ib23.122 | NH | =O | | NCH₃ |
| Ib23.123 | NCH₃ | =O | | NH |
| Ib23.124 | NCH₃ | =O | | NCH₃ |
| Ib23.125 | NC₂H₅ | =O | | NH |
| Ib23.126 | NC₂H₅ | =O | | NC₂H₅ |
ainsi que leurs sels utilisables en agriculture.

4. Composé 4-[2-méthyl-3-(4,5-dihydroisoxazol-3-yl)-4-méthylsulfonylbenzoyl]-1-méthyl-5-hydroxy-1H-pyrazole.

5. Sels du composé 4-[2-méthyl-3-(4,5-dihydroisoxazol-3-yl)-4-méthylsulfonylbenzoyl]-1-méthyl-5-hydroxy-1H-pyrazole utilisables en agriculture.

6. Procédé pour fabriquer des dérivés de benzoyle à substitution 3-hétérocyclyle de formule I ou de formule Ib23 selon les revendications 1 à 5, **caractérisé en ce que** l'on effectue l'acylation du pyrazole de formule II, dans laquelle les variables R¹⁶ et R¹⁸ ont les significations données dans les revendications 1 à 5 : 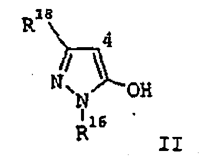 avec un acide carboxylique activé IIIα ou avec un acide carboxylique IIIβ : 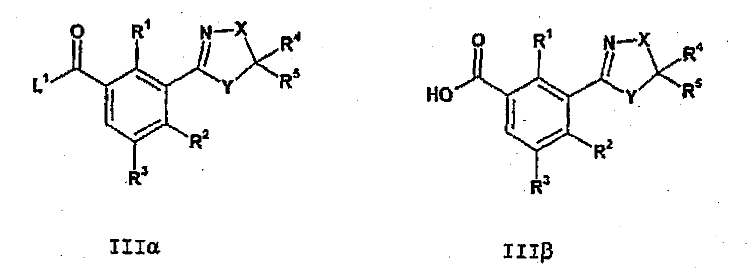 dans lesquels les variables R¹ à R⁵, X et Y ont les significations données dans les revendications 1 à 5 et L¹ représente un groupement partant qui peut être déplacé par un nucléophile, et **en ce que** l'on convertit le produit d'acylation, éventuellement en présence d'un catalyseur, en composé I ou en composé Ib23.

7. Dérivés d'acide benzoïque à substitution 3-hétérocyclyle de formule III : 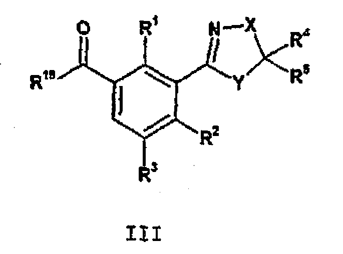 dans laquelle R¹⁹ représente un hydroxyle ou un radical hydrolysable et les variables R¹ à R⁵, X et Y ont les significations données dans les revendications 1 à 5.

8. Dérivés d'acide benzoïque à substitution 3-hétérocyclyle de formule III selon la revendication 7, dans laquelle :
R¹⁹ représente un halogène, un hydroxyle ou un alcoxy en C₁-C₆.

9. Agent contenant une quantité efficace sur le plan herbicide d'au moins un dérivé de benzoyle à substitution hétérocyclyle de formule I ou de formule Ib23, ou d'un sel utilisable en agriculture d'un composé de formule I ou de formule Ib23 selon les revendications 1 à 5, et des adjuvants habituels pour la formulation d'agents phytosanitaires.

10. Procédé pour fabriquer des agents selon la revendication 9, **caractérisé en ce que** l'on mélange une quantité efficace sur le plan herbicide d'au moins un dérivé de benzoyle à substitution 3-hétérocyclyle de formule I ou de formule Ib23, ou d'un sel utilisable en agriculture d'un composé de formule I ou de formule Ib23 selon les revendications 1 à 5, à des adjuvants habituels pour la formulation d'agents phytosanitaires.

11. Procédé pour lutter contre la croissance non souhaitée de plantes, **caractérisé en ce que** l'on fait réagir sur des plantes, leur habitat et/ou sur des semences, une quantité efficace sur le plan herbicide d'au moins un dérivé de benzoyle à substitution 3-hétérocyclyle de formule I ou de formule Ib23, ou d'un sel utilisable en agriculture d'un composé de formule I ou de formule Ib23 selon les revendications 1 à 5.

12. Utilisation des dérivés de benzoyle à substitution 3-hétérocyclyle de formule I ou de formule Ib23 et de leurs sels utilisables en agriculture selon les revendications 1 à 5, comme herbicides.
